# EUROPEAN PATENT SPECIFICATION

(11) **EP 3 119 271 B1**
(45) Date of publication and mention of the grant of the patent: **02.09.2020**
(21) Application number: 15764583.9
(22) Date of filing: 20.03.2015
(51) Int. Cl.: A61B 5/00, A61B 10/00

(54) **HEALTH STATE MONITORING DEVICE**
VORRICHTUNG ZUR ÜBERWACHUNG DES GESUNDHEITSZUSTANDES
DISPOSITIF DE SURVEILLANCE D'ÉTAT DE SANTÉ

(30) Priority: 21.03.2014 US 201461968593 P; 11.04.2014 US 201461978261 P; 16.06.2014 US 201462012668 P; 31.07.2014 US 201462031368 P; 25.09.2014 US 201462055051 P; 26.11.2014 US 201462085206 P; 19.03.2015 US 201514662411
(43) Date of publication of application: 25.01.2017
(73) Proprietor: Razzberry Inc., Berkeley, CA 94710 (US)
(72) Inventor: BARTON-SWEENEY, Alexandra, New Haven, CT 06511 (US)
(74) Representative: Harris, Oliver John Richard
(86) International application number: PCT/US2015/021646
(87) International publication number: WO 2015/143259

(56) References cited:
- WO-A1-03/011142
- WO-A1-2011/038310
- CA-A1- 2 290 898
- US-A1- 2007 249 958
- US-A1- 2009 233 321
- US-A1- 2010 036 212
- US-A1- 2011 098 112
- US-A1- 2012 016 258
- US-A1- 2013 095 459
- US-A1- 2013 137 940

## Description

### CROSS-REFERENCE TO RELATED APPLICATIONS

This application claims the benefit of United States Application Serial No. 14/662,411 filed on March 19, 2015, United States Provisional Application Serial No. 61/968,593 filed on March 21, 2014, United States Provisional Application Serial No. 61/978,261 filed on April 11, 2014, United States Provisional Application Serial No. 62/012,668 filed on June 16, 2014, United States Provisional Application Serial No. 62/031,368 filed on July 31, 2014, United States Provisional Application Serial No. 62/055,051 filed on August 25, 2014, and United States Provisional Application Serial No. 62/085,206 filed on November 26, 2014, the contents of which are incorporated by reference herein in their entirety.

### BACKGROUND OF THE INVENTION

The subject matter disclosed herein relates to a health state monitoring device and in particular to a device that monitors hormonal levels to assist user in achieving or avoiding conception. CA2290898, WO03/011142, US2012/016258 and WO2011/038310 show such devices.

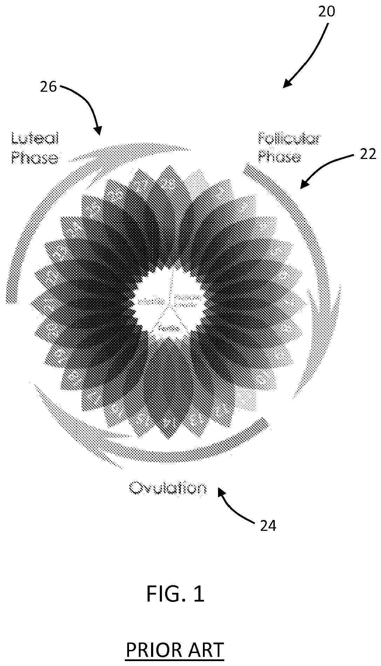

A woman's menstrual cycle consists of a series of regulated hormonal changes that occur in a sequence every month. The cycle 20 shown in FIG. 1 may be divided into three different phases: follicular 22, ovulation 24 and luteal 26 phases. The ability to achieve or avoid conception without external intervention is dependent on the phase of the cycle 20. Typically these cycles have a duration of 22-36 days, with an average cycle being 28 days.

The follicular phase 22 lasts from the first day of a woman's period to the day of ovulation where the lining of the uterus grows to prepare a sperm-friendly environment. Simultaneously, an egg matures in preparation for ovulation. The ovulation phase 24 occurs on or near the middle of the cycle 20. In response to a hormonal surge, the egg is released and awaits fertilization for a period of time, typically 24 hours. The luteal phase 26, lasts from the end of ovulation to the next period. This phase creates an elevation in the hormone progesterone, which increases a woman's basal body temperature (BBT). If fertilization occurs, the egg/embryo is implanted into the endometrium and pregnancy begins. Otherwise the endometrium breaks down leading to menstruation and a start of a new cycle.

A number of different forms of contraception have been developed. Some of these contraception methods either block pregnancy by preventing release of the egg or by creating an unfriendly environment for embryo implantation. These methods have varying degrees of efficacy. For example, oral contraceptives have a failure rate from 0.3% (perfect usage) to 8% (typical usage). While these methods may be effective, as with all medications or surgical procedures there is high risks of side-effects, both in the short term and long term usage (e.g. cancer, heart problems, depression), or complications from surgery (e.g. infection).

Another type of contraception method involves a barrier, such as a condom or a diaphragm. While these methods avoid the complications associated with surgery, there may still be side effects when there is a reaction to the materials used. Further, the failure rates for these methods ranges from 2% - 6% (perfect usage) to 16% (typical usage). It should be appreciated that this failure range results in an appreciable amount of unintended pregnancies.

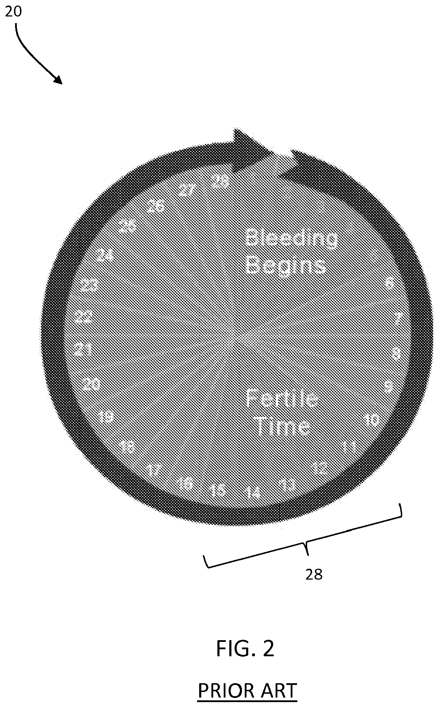

To avoid the complications of the pharmaceutical, surgical, invasive, and barrier techniques, still another type of conception is used that relies on the cyclic nature of the menstrual cycle 20. As shown in FIG. 2, a woman may only conceive during a small window of time 28, between five to six days, which occur before and during ovulation given that sperm can survive up to 5 days while waiting for the release of the egg. A number of techniques (e.g. Natural Family Planning, Standard Days Method, the Rhythm Method) have been used to achieve or avoid conception by allowing a woman to estimate when the fertility window 28 will occur. This is sometimes referred to as fertility awareness based methods (FAB). It should be appreciated that the cycle 20 may vary considerably from woman to woman, and even for a given woman it may change significantly from one cycle to the next. These variations may occur due to changes in hormone levels or environmental factors such as stress. As a result, the efficacy rates can vary significantly.

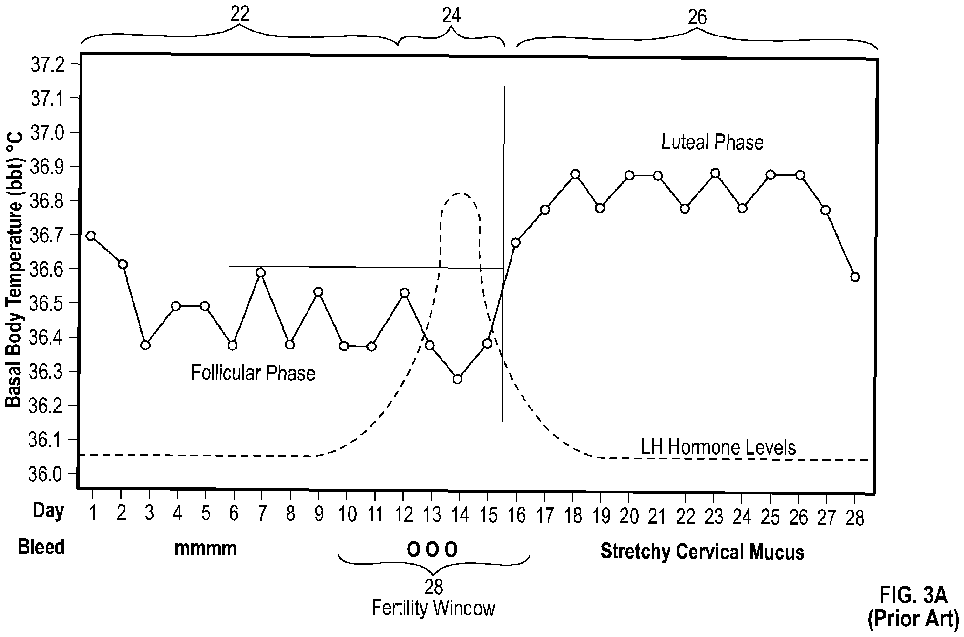

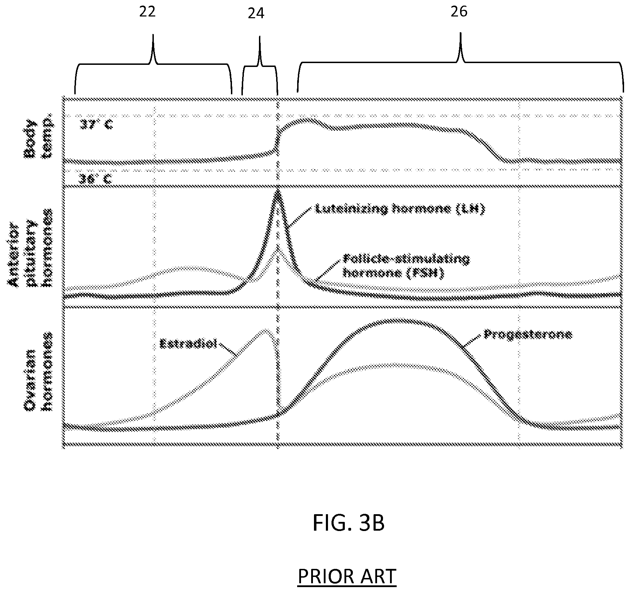

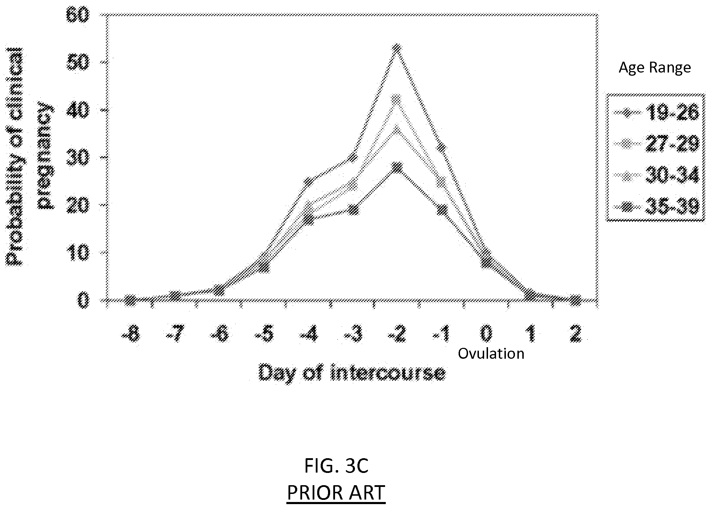

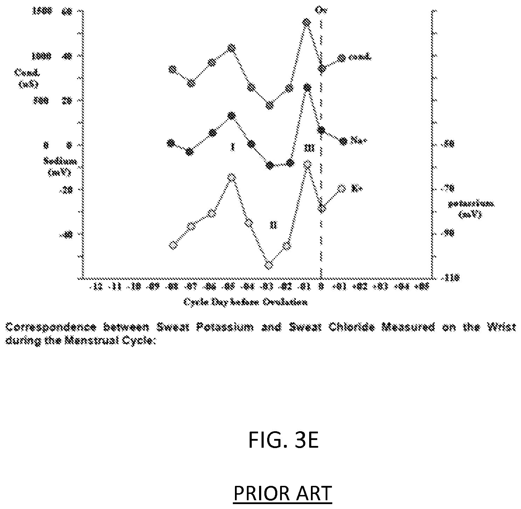

To improve the effectiveness of FAB methods, some systems have been proposed that measure the woman's basal body temperature to assist in determining the phase of the cycle 20. However, as shown in FIG. 3A and FIG. 3B, the woman's temperature increase may not be significant until 3 - 5 days into the fertility window. As a result, the use of basal body temperature by itself is better suited as a predictor for achieving conception rather than avoiding conception. In addition, it cannot always accurately notify users of the fertile window while it is occurring since it measures ovulation after it has occurred and therefore can only predict and not always accurately when a user is in the pre-ovulatory phase. As a contraceptive, it must then overestimate the fertile window with a substantial buffer, thus limiting a user's ability to maximize use of fertile or infertile days. Further, while it has been traditionally thought that conception was at highest probability at ovulation, this is not necessarily the case. As shown in FIG. 3C, it has been found that the probability of pregnancy is highest 2 days prior to ovulation. The level of probability varies depending on age group.

The previously proposed systems have been inadequate in capturing the precise beginning of the fertile window and other events throughout the menstrual cycle, such as peak fertility day, ovulation, menstruation, conception, etc. Traditional approaches such as tracking BBT can only accurately show when ovulation has / is just about to occur since they look for the rise in temperature and do not identify the dip in temperature right before that indicates peak fertility, while systems that track Luteinizing Hormone (LH) are only tracked right after ovulation has occurred since an LH surge occurs at release of the egg. The problem with focusing on ovulation is that once ovulation has occurred, a woman's chances of conception have dramatically decreased to 5%. BBT (FIG. 3C) is also just predictive (25% failure rate) as a contraceptive and is unable to track the beginning of the fertile window. By not tracking the entire fertile window prior to ovulation, a woman loses the opportunity to increase her chances of conception by 80%.

It should be appreciated that achieving or avoiding conception can be time consuming and expensive. It has been estimated that the average woman will spend $10,000 over her lifetime and 200 hours simply on prescription refills and doctors' visits. These estimates do not include the costs and time associated with the treatment of side effects and complications from the contraception methods.

Accordingly, while existing methods of achieving or monitoring a user's health state have been suitable for their intended purposes, the need for improvement remains, particularly in providing a method that is easy and cost effective to use while avoiding complications and side effects.

### BRIEF DESCRIPTION OF THE INVENTION

In accordance with an embodiment, a device for determining a user's physical condition is provided. The device includes a plurality of sensors configured to be carried by a user and measure a plurality of biomarker values associated with the user, each of the plurality of sensors configured to measure a different biomarker value. A controller is operably coupled to the at least one sensors and the indicator, the controller having a processor and memory, the processor configured to execute computer readable instructions when executed on the processor for comparing a data model stored in the memory associated with each of the biomarker values and determining the user's physical condition in response to receiving at least one signal from one of the plurality of sensors, wherein at least one of the data models includes a demographic data model and a personal data model.

In accordance with another embodiment, a method of determining a user's physical condition is provided. The method includes providing a device with a plurality of sensors, each of the sensors configured to measure a biomarker. A plurality of biomarker values are measured on the user with the plurality of sensors. Each of the biomarker values are compared with a population data model associated with that biomarker. Each of the biomarker values is compared with a personal data model. The user's physical condition is determined based on at least one of the measured biomarker values, the population data model and the personal data model.

In accordance with another embodiment, a method for determining a user's physical condition is provided. The method includes providing a device configured to be in contact with the user's skin. The device includes at least one sensor configured to measure a biomarker value. A biomarker is measured on the user. A color of an indicator is changed in response to the measured of the biomarker value crossing a threshold. The physical condition of the user is determined based at least in part on the color of the indicator.

These and other advantages and features will become more apparent from the following description taken in conjunction with the drawings. The invention is defined by the appended claims.

### BRIEF DESCRIPTION OF THE DRAWING

The subject matter, which is regarded as the invention, is particularly pointed out and distinctly claimed in the claims at the conclusion of the specification. The foregoing and other features, and advantages of the invention are apparent from the following detailed description taken in conjunction with the accompanying drawings in which:
FIG. 1 is an illustration of a typical menstrual cycle;
FIG. 2 is another illustration of a menstrual cycle with a fertility window;
FIGS. 3A and 3B are graphical illustration of temperature and hormone biomarker levels as a function of time;
FIG. 3C is a graphical plot of pregnancy probability relative to ovulation date;
FIG. 3D is a graphical plot of changes in sweat chloride concentration during the course of the menstrual cycle;
FIG. 3E is a graphical plot of the correspondence between sweat potassium and sweat chloride measured on the wrist during the course of the menstrual cycle;
FIG. 4 is a side illustration of a fertility monitoring device in accordance with an embodiment of the invention;
FIG. 5 is a graphical plot illustrating saliva composition as a function of menstrual cycle;
FIGS. 6A-6G are a schematic illustration of different embodiments of the fertility monitoring device;
FIG. 7 illustrates a cloud computing node according to an embodiment of the present invention;
FIG. 8 illustrates a cloud computing environment according to an embodiment of the present invention;
FIG. 9 illustrates abstraction model layers according to an embodiment of the present invention;
FIG. 10 is a flow diagram illustrating a method of determining fertility level according to an embodiment of the invention;
FIG. 11 is a block diagram illustrating the artificial intelligence feedback loop for determining fertility level according to an embodiment of the invention;
FIG. 12 is a schematic illustration of a system for determining fertility levels for a group of users having access to single communications device according to an embodiment of the invention;
FIG. 13 is a schematic illustration of another system for determining fertility levels;
FIGS. 14, 15A and 15B are schematic illustrations for another system for determining fertility levels;
FIG. 16 is a graphical plot illustrating eccrine sweat pH as a function of menstrual cycle; and
FIGS. 17A- 17R illustrate exemplary wearable devices incorporating sensors for a fertility monitoring device.

The detailed description explains embodiments of the invention, together with advantages and features, by way of example with reference to the drawings.

### DETAILED DESCRIPTION OF THE INVENTION

Embodiments of the present invention provide for biomarker measurement and an analysis system that provides a powerful approach to identifying both the distinct events in the menstrual cycle as well as fertility level. Embodiments provide for a correlation between a plurality of biomarkers to more accurately and more precisely determine the beginning of the fertile window and other events throughout the menstrual cycle, such as peak fertility day, ovulation, menstruation and conception.

Embodiments of the invention provide for the automatic use of combinations of biomarker measurements at different times during the menstrual cycle to identify different physiological and hormonal events that occur during the menstrual cycle. Embodiments provide for the real-time tracking of fertility related biomarkers at predetermined time periods to provide an improved predictor of a fertility level at a point in time. Embodiments described herein encapsulate different systems and methods for acquiring and analyzing biomarker data to determine fertility level.

Embodiments of the present invention track biomarkers and perform an analysis that can more accurately reflect these menstrual cycle events including the beginning of the fertile window, which is desirable to allow the use as a contraceptive, without requiring a large buffer of time to be effective as well as determining the fertile days prior to ovulation to increase chances of conception by 80%, while 26% of this increase is from tracking the days prior to peak fertility. Prior art system that utilize BBT thermometers that are coupled with computers increase their efficacy by over-estimating the fertile window by an additional 3 - 6 days, this means the woman has to abstain or use another form of contraceptive for a larger portion of her menstrual cycle to lower the risk of pregnancy during this window of time. The BBT thermometer approach makes determining the beginning of the fertile window difficult. Embodiments described herein use biomarkers that have been shown to change in patterns at the beginning of the fertile window in order to capture the entry into the fertile window when it is actually occurring. This means a large buffer of extra days would not be necessary in order to be effective for use as a contraceptive and to remove extraneous days and efforts for couples trying to conceive. Further, these prior art approaches tend to work well in a controlled laboratory setting or require strict adherence to timing usage manually, such as taking temperature immediately upon waking, but become unreliable once real-world factors, such as environmental factors are present. Coupled with analysis and pattern recognition, embodiments of the present invention are better able to predict the fertile level at any point in time as well as remove irrelevant values that do not pertain to the pattern but instead are influenced by other factors that may not be related to the hormonal influences being tracked. The recognition of irrelevant values or values that are influenced by other factors have been a major obstacle for prior art that has tried to detect fertility based only on thresholds and simple ratiometric analysis. The prior art further doesn't take into account other non-hormonal factors that could influence the biomarker values or trend profiles.

Embodiments of the present invention provide for a fertility and hormonal monitoring device that provides a fertility level indication to a user with a high level of efficacy without the use of pharmaceuticals or surgical procedures. Embodiments of the present invention provide fertility level indication based on the measurement of multiple physiologic factors, environmental factors, or biomarkers. Embodiments of the present invention provide for a fertility level indication based on a combination of biomarkers in combination with the user's menstrual cycle history. Embodiments of the present invention provide for a cloud based computing environment for the determination of fertility level. Embodiments of the present invention provide for utilizing artificial intelligence methods for determining a user's fertility level. Still further embodiments of the invention provide for fertility level indication for groups of users having limited communications capabilities, such as in remote villages. It should be appreciated that these embodiments provide advantages in allowing a user to know their fertility level using a low cost easy to use device with a high level of confidence without the side effects or complications of pharmaceutical, invasive, or surgical methods.

It should be appreciated that while embodiments herein describe the determination of a fertility level, this is for exemplary purposes and the claimed invention should not be so limited. In other embodiments, the embodiments of the invention determine hormonal levels to monitor for other medical conditions or status. Further, while embodiments described herein may refer to use of the device by a woman for achieving or avoiding conception, this is for exemplary purposes and the claimed invention should not be so limited. In other embodiments, the user may be male or female and in some application, the embodiments may be used with other mammals to monitor their current state or condition.

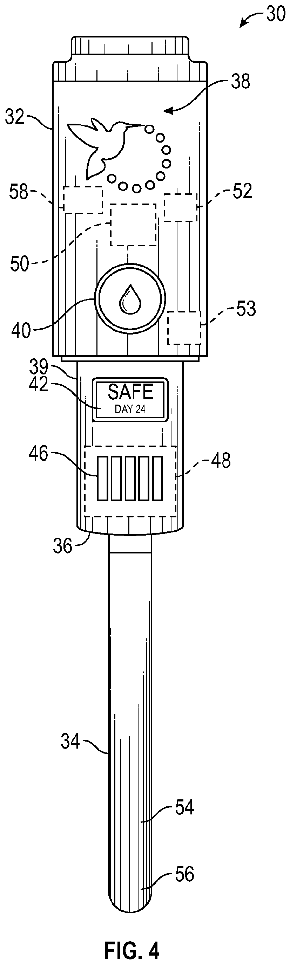

Referring now to FIG. 4, an exemplary embodiment is shown of a fertility monitoring device 30. The device 30 includes a body 32 having a probe 34 extending from an end 36. A counter indicator 38 is arranged in the housing 32 adjacent an actuator or button 40. In other embodiments, the indicator 38 may be disposed in other locations, such as on a side opposite the button 40 or on an end of the housing for example. Further, the button 40 may also be disposed in other locations, such as on the intermediate portion 39 or on the end of the housing 32. The indicator 38 is composed of a plurality of light emitting components, such as light emitting diodes (LEDs). As will be discussed in more detail below, the indicator 38 provides feedback to the user on the status of the measurements, like a timer for example, and also changes color to indicate the user's fertility status. The actuator 40 functions as a user input and allows the user to interact with the device 30.

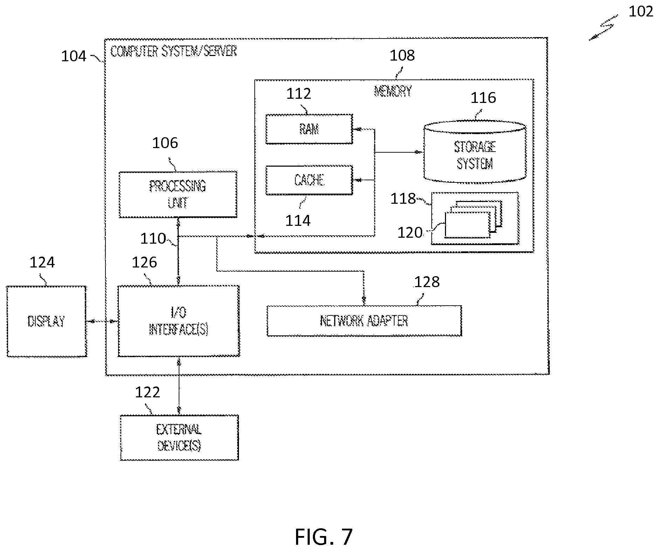

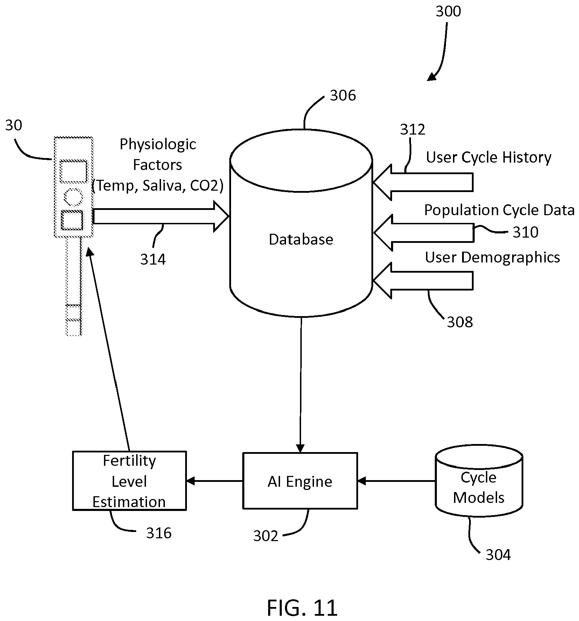

In the exemplary embodiment, the housing 32 includes an intermediate stepped portion 39 arranged between the button 40 and the probe 34. The intermediate portion 39 is sized to receive an opening 45 (FIG. 11) within the cover 44 (FIGS. 7 - 13) that extends over the probe 34 and intermediate portion 39. The intermediate portion 36 may also include a display 42, such as a liquid crystal display (LCD) for example, that allows the device 30 to communicate information, such as but not limited to fertility status, day of the cycle, battery level, time, date, alarm and period day for example. Arranged between the display 42 and the end 36 is a series of vents 46 that allow air breathed by the user to interact with a sensor 48. In the exemplary embodiment, the sensor 48 is a carbon dioxide (CO2) sensor. In one embodiment, the CO2 sensor 48 is an infrared spectrometry device. In one embodiment, the CO2 sensor 48 may be a nondispersive infrared sensor (NDIR) that includes an infrared source, a light tube, an interference (wavelength) filter, and an infrared detector. The gas is pumped or diffuses into the light tube and the electronics measures the absorption of the characteristic wavelength of light. NDIR sensors are most often used for measuring carbon dioxide. In one embodiment, the CO2 sensor 48 may be a Model CO2F-W high speed carbon dioxide sensor manufactured by SST Sensing Ltd. of Coatbridge Scotland for example. In another embodiment, the sensor 48 measures levels of volatile sulfur in the user's breath. CO2 levels and volatile sulfur levels in a user's breath have been shown to vary in correlation with the menstrual cycle. The sensors may also be a sensor formed using a nanotechnology.

In one embodiment, the housing 32 may also include an accelerometer 53 or other sensor that measures motion. The detection of motion may allow the determination if the user is at rest or is active, which in turn may allow for compensation of the readings or the determination of the user's fertility level. In another embodiment, the device 30 may include a sensor for measuring the user's heart rate (i.e. a pulse oximeter or an electrocardiogram sensor) to determine the user's current physical or activity state.

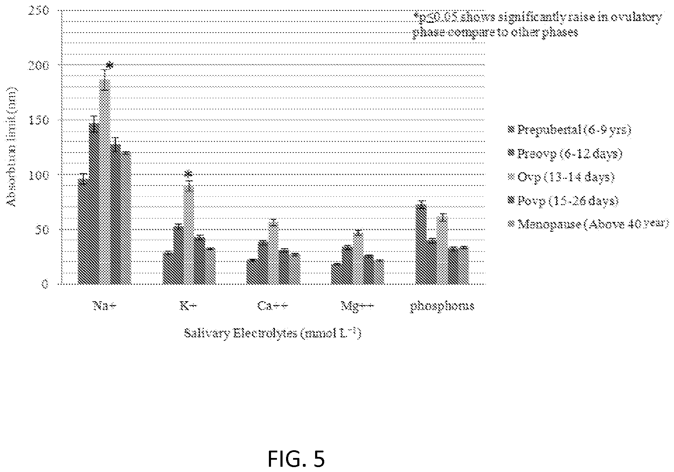

The device 30 may further include a plurality of sensors within the probe 34. In the exemplary embodiment, these sensors include a basal body temperature (BBT) sensor 54 and a saliva sensor 56. The BBT sensor 54 is a measure of the lowest temperature attained by the body during rest (usually during sleep). It is generally measured immediately after awakening and before any physical activity has been undertaken. The saliva sensor 56 measures one or more electrolytes or ions in the user's saliva that have been identified as correlating with the menstrual cycle. In one embodiment, the saliva sensor may detect a compound such as dodecanol. These electrolytes include chloride, sodium, potassium, inorganic phosphorus, magnesium and calcium. As shown in FIG. 5, the levels of these constituents of the user's saliva are elevated during the ovulation phase. Many of these compounds, e.g. chloride and pH, exhibit similar patterns with a surge at the beginning of the fertile window, a fall, and then a second surge 1 - 2 days prior to ovulation near peak fertility. In one embodiment, the saliva sensor 56 is a salinity sensor that determines salinity based on the density and electrical conductivity of the user's saliva. In one embodiment, the salinity sensor may be a sensor such as digital conductivity/salinity sensor RS 485/SDI 12 manufactured by Ponsel Measure Groupe of Caudan, France. In still another embodiment, the probe 34 includes a pH sensor.

The display 42, CO2 sensor 48, BBT sensor 54 and saliva sensor 56 are coupled to communicate with a controller 50 arranged within the housing 32. Controller 50 is a suitable electronic device capable of accepting data and instructions, executing the instructions to process the data, and presenting and storing the results. Controller 50 may accept instructions through button 40, or through other means such as but not limited to electronic data card, voice activation means, manually-operable selection and control means, radiated wavelength and electronic or electrical transfer. Therefore, controller 38 includes a processor that is responsive to computer executable instructions. The controller 38 may further include memory (random access memory, non-volatile memory, read-only memory), one or more input/output (I/O) controllers and a data communications module 52. In the exemplary embodiment, the controller 52 and the components of the device 30 are powered by an energy source 58, such as a rechargeable battery for example. The battery may also be a photovoltaic battery, a silicon nanowire battery or a self-charged graphene battery. A self-charged graphene battery is one that harvests electrical energy from the thermal energy of the environment via ionic thermal motion. In one embodiment, the device 30 measures the physiological parameters/factors simultaneously or substantially simultaneously. This measurement of the physiological parameters/factor may be performed in a single action by the user (e.g. inserting the probe 34 into their mouth).

The communications module 52 allows the controller 38 to communicate with one or more external computer networks. The communication with the external computer network may be direct or through an intermediary device, such as a cellular phone for example. The connection with the external device or intermediary device may be wired, such as via a universal serial bus (USB) connector or a 6.35 mm, 3.5 mm or 2.5mm headphone connector for example. The connection with the external device or intermediary device may be wireless, such as Bluetooth™, near field communication (NFC), radio frequency identification (RFID), transmission via capacitive coupling or electromagnetic induction, WiGig (IEEE 802.11ad) or Wi-Fi (IEEE 802.11) for example. The communications module may include an antenna. The antenna may be nanoelectronics based integrated antenna, or an on-chip antenna. The antenna may be made from silicon, polymer materials, carbon nanotubes, graphene, superconductors (i.e. superconducting quantum interference devices) or plasmonic devices for example. The antennas may be fabricated by evaporation of metallic films, patterning via photo or electron lithography, or grown epitaxially on a substrate. In other embodiments, the circuitry may be fabricated with an ink solution or paste via a printing process, such as copper ink on paper for example. In other embodiments, the nanoelectronics based antenna may be located separately from the processing circuitry, such as to avoid interference or shielding issues for example.

As will be discussed in more detail below, the communications module 52 allows the device 30 to transmit and receive data from one or more computing devices that allows for the determination of a fertility level of the user. The controller 50 is configured to communicate via well-known computer communications protocols, such as but not limited to TCP/IP (Transmission Control Protocol/Internet(^) Protocol), RS-232, ModBus, and the like. As will be discussed in more detail below, in one embodiment the controller 50 automatically synchronizes or transmits the data acquired by the device 30 to the intermediary device or the external computer network.

In general, controller 50 accepts data from sensor(s), such as CO2 sensor 48, BBT sensor 54 and saliva sensor 56 for example, and is given certain instructions for the purpose of comparing the data from the sensor(s) to predetermined operational parameters. As will be discussed in more detail below, the sensors may also include a thermometer, basal body temperature thermometer, a CO2 level detector, and a saliva sensor. The controller 38 compares the operational parameters to predetermined variances (e.g. expected body temperature, CO2 level, saliva composition) and if the predetermined variance is exceeded, generates a signal indicating that measured readings may be in error. For example, if the temperature measurement is too low, this may indicate that the probe 32 was not properly positioned in the user's mouth. Additionally, the signal may initiate other control methods that adapt the operation of the device 30 such as changing the color or configuration of the indicator 38.

As will be discussed in more detail below, in the exemplary embodiment the device 30 will transmit the measurements from sensors 48, 54, 56 to an external computing device that combines these measurements with other data from the user and clinical databases to determine a fertility level. This fertility level may be high (conception likely), low (conception unlikely) or uncertain (user in a transition region of cycle), or it may be more granular and exhibit the probability of conception, which is of particular interest to those trying to conceive during the fertile window where identification of peak fertile days would be desired. The level may also indicate the likelihood for conceiving a male or female child given that female sperm is more likely to fertilize the egg if intercourse occurs in the first part of the fertile window given the consistency of the cervical fluid and the relative robustness of female sperm, while male sperm is likely to reach the egg first closer to ovulation given their faster speed to swim to the egg. The device 30 receives a signal back from the external computing device and communicates the fertility level to the user via the display 42, the indicator 38, an audible tone or a combination of the foregoing. In one embodiment, the display 42 includes a probability of the gender of a child if conceived at that moment in time based on where the user is in the fertile window. In one embodiment, the determination of probability is based on the Shettles Method. In one embodiment, the controller 50 includes control methods that allow the device 30 to directly determine the user's fertility level from the measurements combined with data stored in memory without communicating with an external device or computer network. In still another embodiment, the controller 50 cooperates with a processor on an external device, such as a cellular phone or a laptop computer for example, to determine the user's fertility level without transmitting data to a remote computing device.

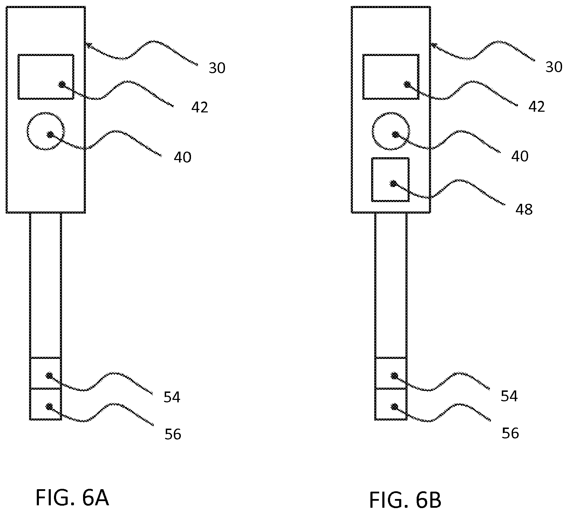

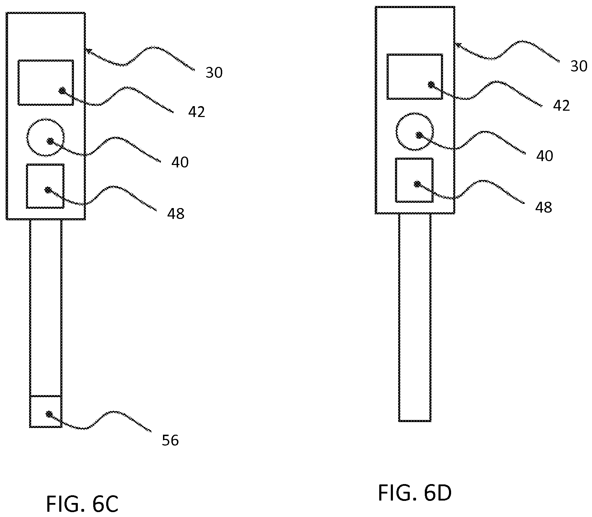

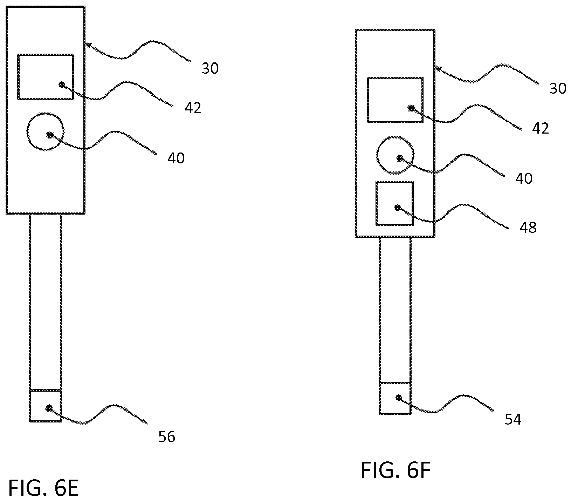

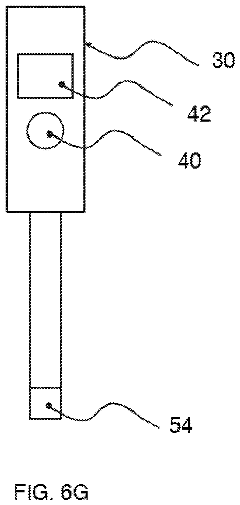

Referring now to FIGS. 6A - 6F, other embodiments are shown of the device 30. In FIG. 6A, the device 30 is shown having a BBT sensor 54 and a saliva sensor 56. It should be noted that in this embodiment, only two physiological factors, basal temperature and saliva composition, are used for determining the fertility level. The device 30 further includes a display 42 and a button/device interaction area 40. The embodiment of FIG. 6B is the same as FIG. 6A with the addition of a CO2 sensor. The embodiment of FIG. 6C is the same as FIG. 6B without the BBT sensor 54. The embodiment of FIG. 6D is the same as FIG. 6C without the saliva sensor 56. The embodiment of FIG. 6E is the same as FIG. 6A with only the saliva sensor 56. The embodiment of FIG. 6F is the same as FIG. 6B without the saliva sensor 56. The embodiment of FIG. 6G is the same as FIG. 6A without the saliva sensor 56.

It should be appreciated that the device 30 may include two or more of the sensors that measure physiological factors that may be combined together to increase the efficacy of the users fertility level. In another embodiment, the device may use one of the sensors if efficacy of the selected sensor is high enough to render other sensors unnecessary.

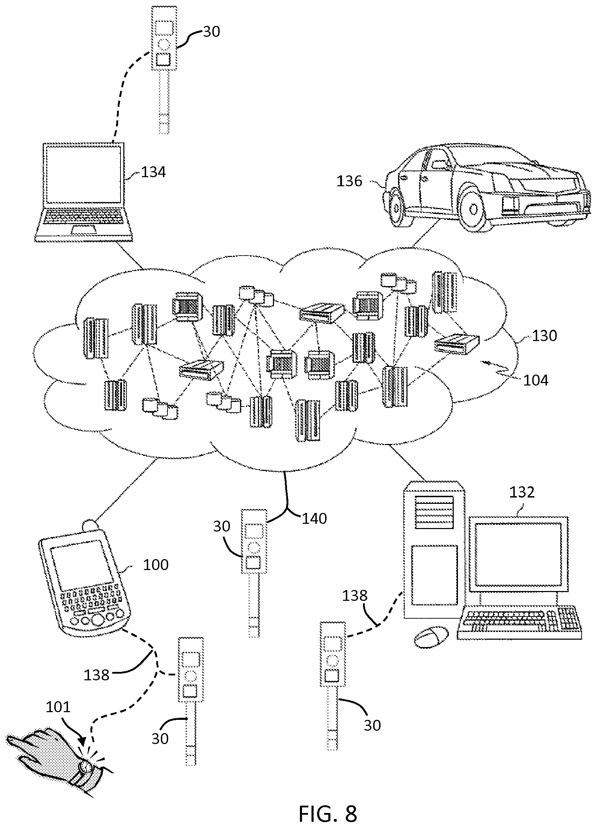

In the exemplary embodiment, the device 30 communicates with a local electronic device, such as a cellular phone 100 (FIG. 8) for example, that provides additional functionality to the user. It should be appreciated that while embodiments herein refer to a cellular phone 100, this is for exemplary purposes and the claimed invention should not be so limited. In other embodiments, the device 30 may communicate with other local external devices, such as but not limited to a wearable device 101 (FIG. 8, e.g. a watch, fitness monitor) or another computing device (e.g. a desktop computer, a laptop, a tablet, appliances or a television) for example. The external wearable device may be for example, glasses having a display that shows the user the data/information from device 30 as described here. The wearable device may also be a watch with a display that shows the user data/information from the device 30. The wearable device may further be an article such as a ring, broach or pendant, that either displays information from the device 30. It should be appreciated that these wearable devices may also indicate or display a subset of the data/information, for example, a ring may have an indicator that changes color based on the users fertility level. The wearable device and other external computing devices each have a processor and memory that is configured to execute computer instructions on the respective processor to perform the functions described herein.

In one embodiment, the cellular phone 100, wearable device and external computing devices may further have one or more applications or control methods that may remind the user (such as by displaying data or information on a screen, actuating a visual indicator or the like) to use the device 30 in the event that data is not received from the device 30, such as on a predetermined schedule for example.

In still other embodiments, the device 30, the cellular phone 100 or the wearable device may cooperate to provide additional information. For example, in one embodiment, the user may provide information from another source, such as through the use or wearing of a fitness or activity monitor that measures user parameters such as temperature, pulse rate, blood oxygen level or blood pressure for example. These activity monitor parameters may be combined or used in place of or in addition to the physiologic parameters/factors measured by the device 30 in the determination of the users fertility level. In one embodiment, the device 30 does not include the BBT sensor 54 and the temperature measurement from the user's activity monitor is used to determine the user's basal temperature.

In one embodiment, the cellular phone 100 includes a processor, a display and a user interface that allows the user to view data acquired by the device 30. For example, the cellular phone 100 may allow the user to view plots or graphs of basal temperature or salinity levels for example. The cellular phone 100 may further display the current fertility level based on that day's measurements. The cellular phone 100 may further provide a predicted fertility level into future/up-coming days based on the current measurements and historical data. The cellular phone 100 may further have control methods that allow the transmission of data and information to external devices. For example, a user may configure the cellular phone to transmit data to a partner for example or allow a user to interact with other users, for example the user can access a support group / forum. In still other embodiments, the cellular phone may include additional control functionality (such as through one or more applications or "apps") for allowing the user to view an analysis of her hormonal / fertility levels / history and any predictions / health trends that may be determined based on data analysis The cellular phone may further have control methods that allow the cellular phone to transmit and receive data from a remote server or group of servers.

It should be appreciated that while embodiments herein illustrate the exemplary embodiment as cooperating with external devices (e.g. cellular phones, network servers), this is for exemplary purposes and the claimed invention should not be so limited. In other embodiments, the device 30 is a standalone device for example.

Referring now to FIGS. 7 - 9, an exemplary computer network 102, sometimes referred to as a "cloud" computer system is shown and described. It is understood in advance that although this disclosure includes a detailed description on cloud computing, implementation of the teachings recited herein are not limited to a cloud computing environment. Rather, embodiments of the present invention are capable of being implemented in conjunction with any other type of computing environment now known or later developed.

Cloud computing is a model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service. This cloud model may include at least five characteristics, at least three service models, and at least four deployment models.

Characteristics are as follows. On-demand self-service: a cloud consumer can unilaterally provision computing capabilities, such as server time and network storage, as needed automatically without requiring human interaction with the service's provider.

Broad network access: capabilities are available over a network and accessed through standard mechanisms that promote use by heterogeneous thin or thick client platforms (e.g., mobile phones, laptops, and PDAs).

Resource pooling: the provider's computing resources are pooled to serve multiple consumers using a multi-tenant model, with different physical and virtual resources dynamically assigned and reassigned according to demand. There is a sense of location independence in that the consumer generally has no control or knowledge over the exact location of the provided resources but may be able to specify location at a higher level of abstraction (e.g., country, state, or datacenter).

Rapid elasticity: capabilities can be rapidly and elastically provisioned, in some cases automatically, to quickly scale out and rapidly released to quickly scale in. To the consumer, the capabilities available for provisioning often appear to be unlimited and can be purchased in any quantity at any time.

Measured service: cloud systems automatically control and optimize resource use by leveraging a metering capability at some level of abstraction appropriate to the type of service (e.g., storage, processing, bandwidth, and active user accounts). Resource usage can be monitored, controlled, and reported providing transparency for both the provider and consumer of the utilized service.

Software as a Service is the capability provided to the consumer is to use the provider's applications running on a cloud infrastructure. The applications are accessible from various client devices through a thin client interface such as a web browser (e.g., web-based e-mail). The consumer does not manage or control the underlying cloud infrastructure including network, servers, operating systems, storage, or even individual application capabilities, with the possible exception of limited user-specific application configuration settings.

Platform as a Service is the capability provided to the consumer is to deploy onto the cloud infrastructure consumer-created or acquired applications created using programming languages and tools supported by the provider. The consumer does not manage or control the underlying cloud infrastructure including networks, servers, operating systems, or storage, but has control over the deployed applications and possibly application hosting environment configurations.

Infrastructure as a Service is the capability provided to the consumer is to provision processing, storage, networks, and other fundamental computing resources where the consumer is able to deploy and run arbitrary software, which can include operating systems and applications. The consumer does not manage or control the underlying cloud infrastructure but has control over operating systems, storage, deployed applications, and possibly limited control of select networking components (e.g., host firewalls).

Deployment Models are as follows. Private cloud: the cloud infrastructure is operated solely for an organization. It may be managed by the organization or a third party and may exist on-premises or off-premises.

Community cloud: the cloud infrastructure is shared by several organizations and supports a specific community that has shared concerns (e.g., mission, security requirements, policy, and compliance considerations). It may be managed by the organizations or a third party and may exist on-premises or off- premises.

Public cloud: the cloud infrastructure is made available to the general public or a large industry group and is owned by an organization selling cloud services.

Hybrid cloud: the cloud infrastructure is a composition of two or more clouds (private, community, or public) that remain unique entities but are bound together by standardized or proprietary technology that enables data and application portability (e.g., cloud bursting for load-balancing between clouds).

A cloud computing environment is service oriented with a focus on statelessness, low coupling, modularity, and semantic interoperability. At the heart of cloud computing is an infrastructure comprising a network of interconnected nodes.

Referring now to FIG. 7, a schematic of an example of a cloud computing node is shown. Cloud computing node 100 is only one example of a suitable cloud computing node and is not intended to suggest any limitation as to the scope of use or functionality of embodiments of the invention described herein. Regardless, cloud computing node 102 is capable of being implemented and/or performing any of the functionality set forth hereinabove.

In cloud computing node 102 there is a computer system/server 104, which is operational with numerous other general purpose or special purpose computing system environments or configurations. Examples of well-known computing systems, environments, and/or configurations that may be suitable for use with computer system/server 104 include, but are not limited to, personal computer systems, server computer systems, thin clients, thick clients, hand-held or laptop devices, multiprocessor systems, microprocessor-based systems, set top boxes, programmable consumer electronics, network PCs, minicomputer systems, mainframe computer systems, and distributed cloud computing environments that include any of the above systems or devices, and the like.

Computer system/server 104 may be described in the general context of computer system-executable instructions, such as program modules, being executed by a computer system. Generally, program modules may include routines, programs, objects, components, logic, data structures, and so on that perform particular tasks or implement particular abstract data types. Computer system/server 104 may be practiced in distributed cloud computing environments where tasks are performed by remote processing devices that are linked through a communications network. In a distributed cloud computing environment, program modules may be located in both local and remote computer system storage media including memory storage devices.

As shown in FIG. 7, computer system/server 104 in cloud computing node 102 is shown in the form of a general-purpose computing device. The components of computer system/server 104 may include, but are not limited to, one or more processors or processing units 106, a system memory 108, and a bus 110 that couples various system components including system memory 108 to processor 106.

Bus 10 represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnects (PCI) bus.

Computer system/server 104 typically includes a variety of computer system readable media. Such media may be any available media that is accessible by computer system/server 104, and it includes both volatile and non-volatile media, removable and non-removable media.

System memory 108 can include computer system readable media in the form of volatile memory, such as random access memory (RAM) 112 and/or cache memory 114. Computer system/server 104 may further include other removable/non-removable, volatile/non-volatile computer system storage media. By way of example only, storage system 116 can be provided for reading from and writing to a non-removable, non-volatile magnetic media (not shown and typically called a "hard drive"). Although not shown, a magnetic disk drive for reading from and writing to a removable, non-volatile magnetic disk (e.g., a "floppy disk"), and an optical disk drive for reading from or writing to a removable, non-volatile optical disk such as a CD-ROM, DVD-ROM or other optical media can be provided. In such instances, each can be connected to bus 110 by one or more data media interfaces. As will be further depicted and described below, memory 108 may include at least one program product having a set (e.g., at least one) of program modules that are configured to carry out the functions of embodiments of the invention.

Program/utility 118, having a set (at least one) of program modules 120, may be stored in memory 108 by way of example, and not limitation, as well as an operating system, one or more application programs, other program modules, and program data. Each of the operating system, one or more application programs, other program modules, and program data or some combination thereof, may include an implementation of a networking environment. Program modules 120 generally carry out the functions and/or methodologies of embodiments of the invention as described herein.

Computer system/server 104 may also communicate with one or more external devices 122 such as a keyboard, a pointing device, a display 124, etc.; one or more devices that enable a user to interact with computer system/server 104; and/or any devices (e.g., network card, modem, etc.) that enable computer system/server 104 to communicate with one or more other computing devices. Such communication can occur via I/O interfaces 126. Still yet, computer system/server 104 can communicate with one or more networks such as a local area network (LAN), a general wide area network (WAN), and/or a public network (e.g., the Internet) via network adapter 128. As depicted, network adapter 128 communicates with the other components of computer system/server 204 via bus 110. It should be understood that although not shown, other hardware and/or software components could be used in conjunction with computer system/server 104. Examples, include, but are not limited to: microcode, device drivers, redundant processing units, external disk drive arrays, RAID systems, tape drives, and data archival storage systems, etc.

Referring now to FIG. 8, illustrative cloud computing environment 130 is depicted. As shown, cloud computing environment 130 comprises one or more cloud computing nodes 104 with which local computing devices used by cloud consumers, such as, for example, personal digital assistant (PDA) or cellular telephone 100, desktop computer 132, laptop computer 134, and/or automobile computer system 136 may communicate, and third party devices, such as wearable devices or other external devices. Nodes 104 may communicate with one another. They may be grouped (not shown) physically or virtually, in one or more networks, such as Private, Community, Public, or Hybrid clouds as described hereinabove, or a combination thereof. This allows cloud computing environment 130 to offer infrastructure, platforms and/or software as services for which a cloud consumer does not need to maintain resources on a local computing device. It is understood that the types of computing devices 100, 132, 134, 136 shown in FIG. 8 are intended to be illustrative only and that computing nodes 104 and cloud computing environment 130 can communicate with any type of computerized device over any type of network and/or network addressable connection (e.g., using a web browser).

As shown in FIG. 8, the device 30 may be connected to the computing nodes 104 via the cloud computing environment 130, either through an intermediary device 100, 132, 134, 136. The connection from the device 30 to the intermediary device 100, 132, 134, 136 is through a communications medium 138 that may be wired or wireless as described herein above. In one embodiment, the device 30 is connected directly to the cloud computing environment 130 via a wired or wireless communications medium 140.

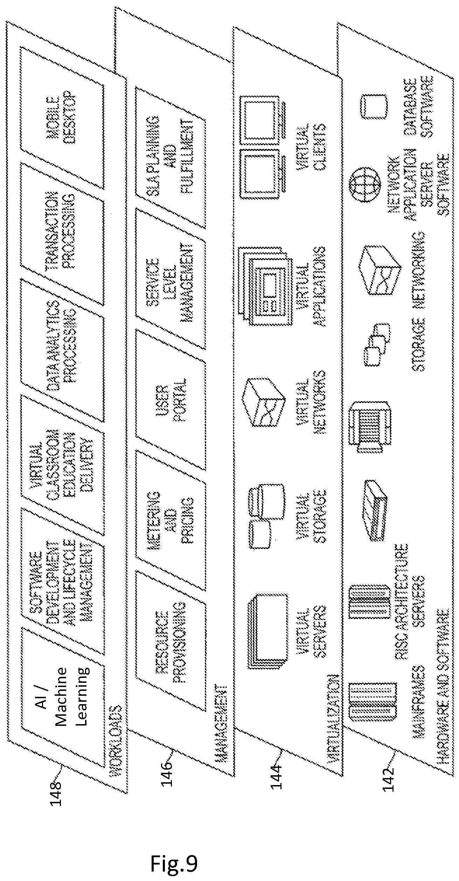

Referring now to FIG. 9, a set of functional abstraction layers provided by cloud computing environment 130 (FIG. 8) is shown. It should be understood in advance that the components, layers, and functions shown in FIG. 9 are intended to be illustrative only and embodiments of the invention are not limited thereto. As depicted, the following layers and corresponding functions are provided:

Hardware and software layer 142 includes hardware and software components. Examples of hardware components include mainframes; RISC (Reduced Instruction Set Computer) architecture based servers; storage devices; networks and networking components.

Virtualization layer 144 provides an abstraction layer from which the following examples of virtual entities may be provided: virtual servers; virtual storage; virtual networks, including virtual private networks; virtual applications and operating systems; and virtual clients.

In one embodiment, one or both of the hardware and software layer 142 and the virtualization layer 144 may include edge components, such as a web server front end and image cache, as well as an image library store, e.g., in a high- performance RAID storage area network (SAN).

In one example, management layer 146 may provide the functions described below. Resource provisioning provides dynamic procurement of computing resources and other resources that are utilized to perform tasks within the cloud computing environment. Metering and Pricing provide cost tracking as resources are utilized within the cloud computing environment, and billing or invoicing for consumption of these resources. In one example, these resources may comprise application software licenses. Security (not shown) provides identity verification for cloud consumers and tasks, as well as protection for data and other resources. User portal provides access to the cloud computing environment for consumers and system administrators. Service level management provides cloud computing resource allocation and management such that required service levels are met. Service Level Agreement (SLA) planning and fulfillment provides pre- arrangement for, and procurement of, cloud computing resources for which a future requirement is anticipated in accordance with an SLA.

Workloads layer 148 provides examples of functionality for which the cloud computing environment may be utilized. Examples of workloads and functions which may be provided from this layer include: artificial intelligence and machine learning modules for predicting fertility levels; software development and lifecycle management; virtual classroom education delivery; data analytics processing; transaction processing; and a mobile desktop for intermediary devices (e.g., 100, 132, 134, 136, as well as mobile nodes 102 in cloud computing environment 130) accessing the cloud computing services.

As will be discussed in more detail below, the cloud computing environment 130 provides advantages in allowing the use of artificial intelligence and machine learning engines to process data from the device 30. The cloud computing environment 130 further provides advantages in making data available to third parties, such as partners, doctors, clinics or other medical providers to allow for care or treatment of the user. In one embodiment, the cloud computing environment 130 provides still further advantages in allowing for the collection of anonymous data on women's menstrual cycles that may be aggregated (user aggregate data) and stored for use by the artificial intelligence and machine learning engines. This data can also be used and mined to extrapolate trends that may enhance usage or provide other uses for the device. This analysis, learning, extrapolation and trending of the data may be performed on a real time basis.

In the exemplary embodiment, the device 30 connects to the cloud computing environment 130 via an intermediary device such as cellular phone 100 or a tablet device for example. This connection of the device 30 with the cloud computing environment 130 allows for two way communications between the device 30 and the cloud computing environment 130. In this embodiment, the functionality of the system may be distributed between the device 30, the cellular phone 100 (intermediary device) and the cloud computing environment 130. This improves the user experience by presenting information and data on the device that is appropriate to display the information. Further, the device 30 may update the cloud computing environment 130 with user data (e.g. basal temperature, salinity and CO2 measurements), and the cloud computing environment 130 may update the cellular phone 100 with real-time data from other user's data to improve the artificial intelligence/machine-learning control methods in the cellular phone 100 and/or device 30. This provides advantages in providing the device 30 with updated artificial intelligence / calculations that are continually improved on a real-time basis rather than just using historical data.

This arrangement provides still further advantages in having the user's data backed up to the intermediary device (cellular phone 100) and to the cloud computing environment 130. It should be appreciated that in addition to preventing loss of data in the event the device 30 or cellular phone 100 is lost or damaged, this configuration allows for the sharing of information and data, such as with the user's doctor or partner for example. Still further, this configuration provides advantages in allowing the data to be retrieved on any platform (e.g. operating system) capable of connecting to the cloud computing environment 130, thus allowing the user to view the data even if the device 30 is in a different location.

This configuration still further allows the analysis and trending of the user's data, either by itself or in combination with other user's data. Thus a comparison of the user's data with that of other user's may be performed on a real time basis. This analysis and trend information may then be displayed to the user via applications on the cellular phone 100. In one embodiment, the aggregate user data may be weighted such that some user's data (e.g. long time users) has a greater impact on the analysis than other user's (e.g. short-time users).

In still another embodiment, the cloud computing environment 130 allows the user to connect with a community of other users, such as via an application on the cellular phone 100. This provides advantages to the user in allowing them to connect via an electronic medium to share information and collaboratively help other uses regarding health or fitness matters.

Further, in still other embodiments, the device 30, the intermediary device (cellular phone 100) and cloud computing environment 130 may be configured to transmit and receive, sometimes referred to push or pull, the user data or user aggregate data automatically between the device 30, the intermediate device and the cloud computing environment.

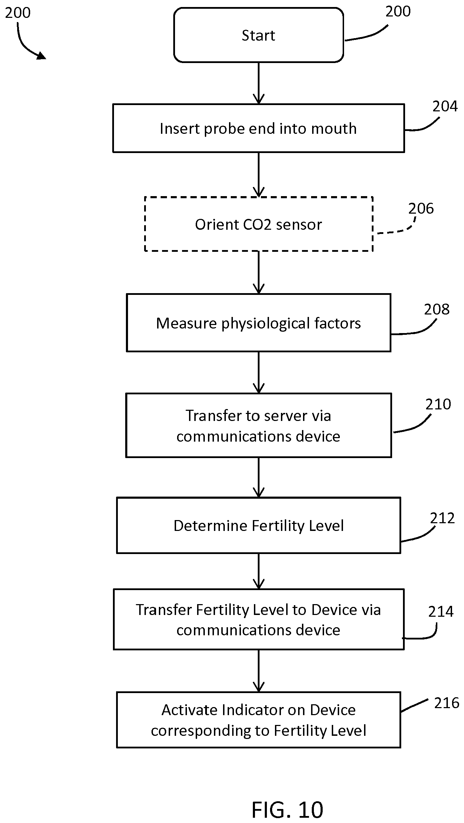

Referring now to FIG. 10, an embodiment is shown of a method for operating the device 30. The method 200 starts in block 202, typically but not limited to when the user first wakes up in the morning. This depends on the sensor combination which may or may not occur upon awakening or at the same time daily. The method 200 then proceeds to block 204 where the user inserts the probe 34 into their mouth to start the physiologic measurements. In one embodiment, the insertion of the probe 34 into their mouth automatically initiates the measurements (e.g. detecting the change in temperature). In another embodiment, the measurements are initiated by a user action, such as depressing the button 40 for example. In the exemplary embodiment, the device 30 provides feedback to the user on the status of the measurements, such as with indicator 38 for example or an audio tone. An optional step 206 may be included for orienting the vents 46 to allow CO2 from the user's exhaled breath to reach the sensor 48. The process 200 then measures the physiologic factors (e.g. temperature, salinity, CO2, pH, ions) in block 208. In one embodiment, the device 30 triggers an alarm (audio or visual) prompting the user to readjust the position of the device 30 or to wake up or use the device at a pre- determined time daily which is set by the user beforehand. In one embodiment, directions on how to readjust are displayed on display 42.

In embodiments using one or more external devices (e.g. cellular phone 100, cloud computing environment 130), the process transmits the measured data, along with any other data input by the user on the device 30 (e.g. start of menstruation) to the external device in block 210. The user's fertility level is determined in block 212. As will be discussed in more detail below, in the exemplary embodiment, the determination of fertility level is performed with the assistance of artificial intelligence/machine-learning engines that combine multiple datasets with the measured data to ascertain the user's fertility level. The determined fertility level is transmitted back to the device 30 in block 214. The device 30 then indicates to the user in block 216 their current fertility level.

The indication of fertility level to the user may take one or more forms, including but not limited to: changing the color of the indicator 38; displaying a message on the display 42 or emitting an audible sound for example. The change of color may be defined based on level of risk of conception, e.g. red for high risk, green for low risk or yellow when the risk is uncertain as well as additional colors representing cycle events such as ovulation, peak fertile day, and menstruation. In other embodiments, the fertility level may be a numerical value or textual.

The fertility level may be determined using several methods. In one embodiment, the biomarkers (e.g. temperature, salinity, pH, skin thickness CO2) may be compared to predetermined thresholds. In other embodiments, the biomarkers are tracked over time and compared against predetermined data trends or models. When one or more of the biomarkers crosses a predetermined threshold or exhibits a predetermined trend then the fertility level associated with that threshold is indicated. In another embodiment, when two or more factors cross a threshold or exhibit a predetermined trend, then the risk is determined to be high, while if only one factor crosses a threshold or trend then the risk would be classified as uncertain. In still another embodiment, the threshold determination is combined with the day of the user's current cycle or with other user input (e.g. cervical fluid observations, Basal Body Temperature (BBT)). The determination of the threshold level or the trend profile may be based on general population data models, historical data models for the individual, or a combination of both. As used herein, a tracked parameter "crosses" a threshold if the value of the parameter rises above (e.g. exceeds) or falls below the threshold.

As used herein the term "data model" includes any known data model for analyzing measured parameters, such as but not limited to a mathematical representation of collected data against which measured values may be compared. The data model may be in the form of a mathematical expression such as a line (e.g. a polynomial curve) fitted to the data or may be a database of values for example. As used herein the term "population data model" is a mathematical representation of collected data for a large group of individuals. In one embodiment, the population data model may be trained over time as additional user data is collected and stored. In one embodiment, the data model may be trained using other data such as from either other demographic groups or a historical data that is directed at different demographic groups to prepopulate the data model to provide the desired accuracy level prior to the availability of large amounts of user data. In one embodiment, the population data model may represent an average value for the group measured. In one embodiment, the population data model may represent a target demographic of individuals, such as those shown in FIG. 3C where the collected data is segregated by age. The population or demographic data model may also represent expected biomarker values for a normal healthy individual. As used herein, the term "personal data model" is a mathematical representation of historical data for the particular user wearing the device. The biomarker values measured by the device may be compared to both the population data model and the personal data model when determining a fertility status. In embodiments where multiple users use the same device, the device may be configured to determine a personal data model based on the user inputting a PIN or password.

The comparison of biomarker values to data models may include comparing absolute biomarker values or a relative change in the biomarker values. This can include comparisons in relation to their expected values and data models, as well as each others values and data models. In one embodiment, the comparison of biomarker values to the data models includes comparing a profile trend of biomarker values measured over a plurality of time periods, such as over the course of several days for example, with the data model. The comparison of the profile trend may include profiles such as a rapid increase in BBT followed by a rapid decline in measured values for example, or may include a rapid increase in skin thickness for example.

In one embodiment, the measured values are stored in memory and the personal data model is updated to include these stored values. The updating of the personal data model may be on a periodic or aperiodic basis. In one embodiment, the personal data model is updated in real time. As discussed herein, the measured values may be stored and combined with those from other individuals and incorporated into the population data model.

Referring now to FIG. 11, an exemplary embodiment is shown of a feedback loop system 300 for determining the user's fertility level. It should be appreciated that the system 300 may be comprised of one or more computer nodes 102 in the cloud computer environment 130 or may also be directly calculated on the device. The system 300 includes an artificial intelligence (or machine learning) engine 302 and women's cycle models 304 that are learned and trained on system data stored in a database 306 (e.g. an SQL server database). The data on which the models are learned and updated includes both static data, such as the user's demographic data 308 or population cycle data 310 for example, and dynamic data, such as the user's cycle history 312 (including cycle length and phase length) or their medical history for example. Static data refers to data which is to be applied over a long term (e.g., the current year or season), and dynamic data refers to data that is to be applied or is applicable to a short term interval (e.g., minutes, hours, or days) around the time of an event (e.g., menstruation or pregnancy event). Dynamic data further includes the current biomarker or physiologic data 314 that is transmitted by the device 30.

The artificial intelligence (AI) engine 302 combines the data 308, 310,312, 314 and the modules 304 to determine an estimated fertility level 316. The AI engine 302 may use, but is not limited to the following AI approaches: time series analysis (linear and nonlinear), Markov models, Hidden Markov models, regression and time series prediction, statistical tests, independent component analysis, decision tree learning; association rule learning; artificial neural networks; inductive logic programing; support vector machines; clustering; Bayesian networks, reinforcement learning; representation learning; similarity and metric learning; and, sparse dictionary learning for example. Once the fertility level 316 is estimated, a signal is transmitted back to the device 30 (or the intermediary device 100) so the user will know their current status. In another embodiment, the device 30 may not include an indicator to notify the user of their current status. In this embodiment, the user's current status may be communicated via a secondary or intermediary device, such as device 100 for example.

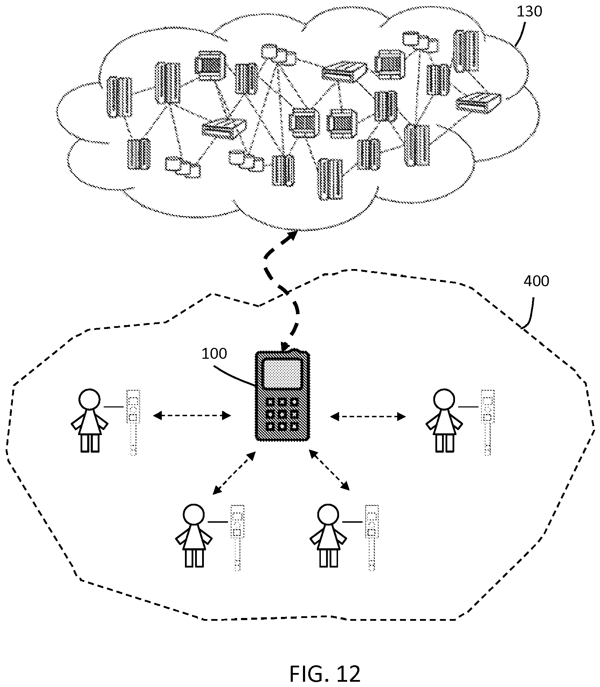

Embodiments described herein reference a single device 30 and intermediary device 100 that allows the user to communicate with the cloud computer environment 130. However, in some applications, such as in developing countries with remote population centers (e.g. villages) or where tracking the fertility of multiple mammals is necessary, for example on cattle farms, this configuration may not be practical due to a lack or limited communications infrastructure. In these applications, the ability for users to share a single intermediary device 100 or share a device 30 may be more suitable. In one embodiment, shown in FIG. 12, a group of users 400 in a commonly located area utilize a single cellular phone to communicate with the cloud computing environment 130. In this embodiment, the device 30 or the software application on the cellular phone 100 may provide a user interface for the user to input their identification, such as a username, UUID, personal identification number (PIN) or password, so that the AI engine can utilize the user's information in the fertility level determination. In another embodiment, the user's identity is not provided and the AI engine uses population cycle data for the fertility level determination. In still another embodiment, a single device 30 is shared among the group of users 400.

Similarly, the device 30 or the cellular phone 100 may provide for the user to input a unique identifier to allow the system to associate the measurements with the user's data. In another embodiment, the users each have a device (e.g. wearable device) that connects to a shared secondary device. The user's information is then identified based on an id associated with the user's device or with syncing with her device. The user can then receive information on her status despite having a common secondary device.

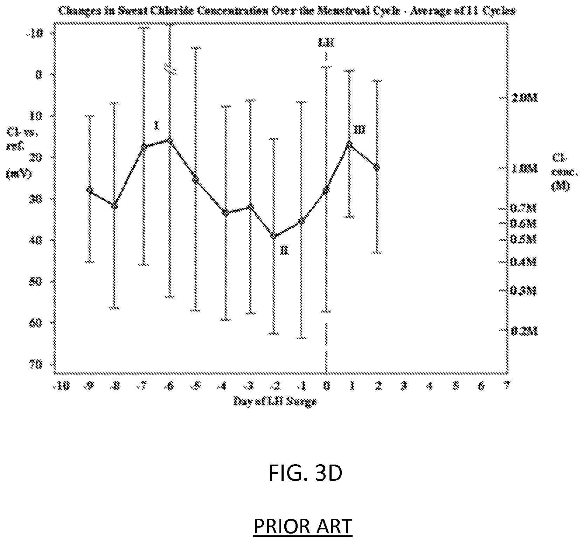

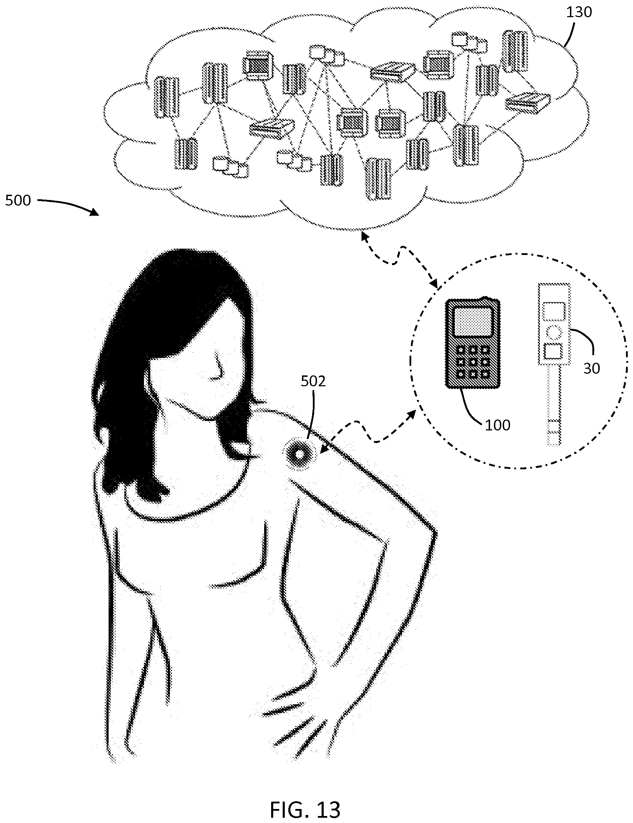

Referring now to FIG. 13, another embodiment is shown of a fertility monitoring system 500 incorporating multiple sensors for determining a fertility status. In this embodiment, a sensor device 502 is arranged in direct contact with the user's skin to measure various biomarker changes (e.g. biochemical / thermal) that are significant to hormonal changes such as the menstrual cycle. These biomarker changes include at least one of the following measurements: body temperature, skin humidity, blood flow, pH, ion concentration in eccrine sweat (one or more ions - can be used to detect the fertile window for instance the chloride surge is one of the earliest indicators of the fertile phase, FIG. 3D-3E), apocrine sweat, temperature, water retention / skin thickness, blood flow, impedance, lipid changes, CO2, lactic acid/lactate system, free amino acid secretion, androstenedione, beta-human chorionic gonadotrophin (hCG), and CO2 bicarbonate. In one embodiment, the sensors may include a sensor similar to that described in United States Patent Publication 2013/0197319 entitled "Flexible Electrode for Detecting Changes in Temperature, Humidity, and Sodium Ion Concentration in Sweat," the contents of which are incorporated herein by reference. In one embodiment, the skin thickness may be measured using echo density, ultrasound or piezo electricity to determine a skin thickness value, which has been shown to vary depending on the phase of the menstrual cycle.

In one embodiment, the concentrations of at least two ions from the eccrine sweat are measured and compared. In one embodiment, at least one ion is selected from the group consisting of potassium (K+), ammonium (NH4+), calcium (Ca2+), chloride (Cl-), nitrate (NO3-) and sodium (N a+). The concentrations of the ions or the other physiologic parameters (e.g. body temperature, skin humidity, blood flow, pH) may be measured simultaneously, consecutively, or at multiple time periods. In one embodiment, the measurements of physiologic parameters are performed in a pattern of predetermined time periods.

One advantage of the present invention is that changes in concentrations of several different types of ions in eccrine sweat can be sensed and analyzed to predict ovulation and other events in the menstrual cycle, mostly due to the hormonal changes that occur throughout the cycle, in female mammals such as human females. These ions include sodium (Na+), potassium (K+), ammonium (NH4+), calcium (Ca2+) and nitrate (NO3-). In this way, different types of sensors can be selected to sense the corresponding ions, such as sodium (Na+), chloride (Cl-), ammonium (NH4+), potassium (K+), calcium (Ca2+) and nitrate (NO3-). In addition, sensors to sense the conductivity of eccrine sweat, thereby indirectly measuring the total concentration of all of the ions, can be used. This permits a selection to be made as to which sensor is most reliable for a particular situation or based on the phase of the menstrual cycle.

For instance, in colder climates where the user may excrete less eccrine sweat, a different type of ion, and a different type of sensor, could be used than in warmer climates where more eccrine sweat is excreted. Likewise, in veterinarian use, different sensors to sense different ions could be used depending on the particular situation and mammal whose fertility status is being sensed. Furthermore, this permits the sensor to be selected based on features other than reliability, such as cost and availability. In one embodiment, a fluid transport system, such as but not limited to a wicking material or hydrophilic or moisture absorbent material may be disposed between the sensor and the user's skin to facilitate the transportation of sweat to and away from the sensor.

It should be appreciated that the fluid transport system may provide additional advantages in allowing the collection of fresh (e.g. recent) samples of the fluid being measured. For example, the fluid transport system may allow recently perspired sweat to be measured and avoid measuring sweat from a previous time period. In one embodiment, the device may determine the reliability of the fluid being analyzed by correlating with other factors, such as environmental humidity, environmental temperature or heart rate when sampling sweat for example. In one embodiment, the fluid transport system uses capillary action or a piezo-electric fluid transportation arrangement to move the fluid to the sensor.

A further advantage of the present invention is that it provides for measurement of changes in concentrations of more than one ion in eccrine sweat. In this way, the changes in concentration of two or more ions can be monitored to provide confirmatory readings in order to more accurately predict ovulation and avoid false readings due to non-hormonal effects such as eccrine sweat volume, diet, washing, personal hygiene, exercise, ambient temperature, variation in the location of the patch, and stress.

By analyzing these biomarkers over time a sequential estimator that regularizes the values may be created.

In still another embodiment, the concentrations of one or more compounds from apocrine sweat are measured and compared. In this embodiment, variations in the concentrations of androstenol or dehydropiandrosterone sulfate indicate the onset of the fertile period which allows for the prediction of ovulation. As is discussed in more detail below, the sensor may be adhered to the skin. In this embodiment, the sensor may be placed in an area where apocrine sweat glands are found, namely the axillae area, areola and nipples of the breast, or in the perianal region for example.

It should be appreciated that sweat may also include other compounds, such as but not limited to water, salt (NaCl), electrolytes, Vitamin C, long chain fat molecules and urinic traces. Further, sweat may contain a fluid with proteins, lipids and steroids that could also be measured to deduce health/fertility level. For example, the sensor device may be used to diagnose deficiencies in these fluids that could allow the user to ascertain their health state. In one embodiment, a woman who is trying to become pregnant and is having difficulty may determine that her levels of Vitamin C (cevitamic acis) or oestrone in her sweat are past a particular threshold. It has been found that these physiologic parameters may prevent egg implantation or induce miscarriage.

The wearable sensor device 502 may be a patch or band such as a printed electronic skin tattoo or electronic patch for example that adheres to the skin temporarily or can be a wearable device such as a garment or accessory that is close to the skin, such as underwear or an armband. In this embodiment, the sensor device 502 may be similar to that described in PCT Application WO 2014/025430 entitled "Wearable Electrochemical Sensors," the contents of which are incorporated herein by reference. The sensor device 502 has built-in sensor(s) that collects biomarker values from the skin. A controller may cooperate with these sensors to receive signals from the sensors. The controller may include memory and data communications circuits for storing data and transmitting/receiving signals. The controller may further include analog-to- digital conversion circuits or a data compression module. The measured data may then be sent to either an external device, such as device 30 or cellular phone 100 for example, or may be used to effect / calculate / store a change on the sensor device 502. The sensor device 502 could indicate what the 'change' means on the actual device in the form of some type of display / led / color / symbol change that could be a result of a calculation or merely the result of a chemical change. For example, the color of some material on the sensor device 502 may change to indicate a fertile or non-fertile day, based on sensing for example the chloride ion surge before ovulation or based on a chemical reaction to the pH or ion change. In other embodiments, there may be a memory circuit, or may also include a power source (e.g. rechargeable, silicon nanowire battery, ionic thermal motion type battery, or a rechargeable thin film flexible battery that is made from materials such as zinc anodes and solid-state electrolytes), or the sensor device 502 is a disposable one use (e.g., one day or one week) device that only has sufficient charge for a short time period to make measurements and send transmissions of those measurements. In this embodiment, the power source may not be rechargeable.

The electrode could be formed from a material such as gold or other organic polymer, carbon nanotubes or another material that could reverse its chemical reaction to take consecutive measurements. The printed sensor could also use graphene, superconductors or plasmonic devices. As well as other materials for example, palladium nano-particles for hydrogen detection or enzyme functionalized carbon nanotubes.

The device could be in the form of a temporary tattoo that will be made in some part or all, of materials that would allow the tattoo / circuit / underlying adhesive to biodegrade, dissolve, disintegrate, or fall apart over time just like a real temporary tattoo. The device could also be in the form of a sticker with a circuit embedded into it. The device could then be used for an entire cycle and then disposed of monthly / per cycle (manually or disintegration over time as in a temporary tattoo) or short-term (daily or weekly for example) or as a long-term, non-disposable rechargeable device.

One embodiment of the device may be able to be placed anywhere on the skin of the body.

The device could have a layer above the circuit (or the circuit could be embedded into this layer): that has the primary purpose of being decorative or of covering up the circuit. The sensors incorporated into the circuit may also be any one of the aforementioned nanotechnology sensors. Further, the skin mounted circuit may include radio frequency identification tags (RFIDs), flexible electronic displays, low cost photovoltaics, conformal antennas, and smart active coatings.

In one embodiment, the skin mounted sensor may include an accelerometer or a heart rate monitor to measure the user's motion or heart rate. This allows for the determination of the user's current state, such as whether they are at rest (i.e. a sleep). The activity level of the user may impact the body temperature measurements and the determination of fertility level. For example, if the user's heart rate or the accelerometer indicates the user is at rest, the Basel body temperature measurement may be more considered as having greater reliability.

In one embodiment, the power source could also be in the form of a biobattery, powered by a compound found in sweat or skin secretions (e.g. lactic acid, enzymes, electrolytes), solar energy, or kinetically. Such a biobattery may be the same as that described in PCT Application WO2013/130145A2, the contents of which are incorporated herein by reference in its entirety.

In one embodiment, the biological fluid includes at least one of the following - perspiration, blood, urine, saliva, or lacrimal fluid. In another embodiment, the biological fluid includes at least one of glucose, alcohol, lactic acid, urea, uric acid, or ascorbic acid, follicle stimulating hormone (FSH), estrogen, progesterone, testosterone, androstenedione, beta-human chorionic gonadotriphin (hCG). In one embodiment, the catalyst includes at least one of glucose oxidase, lactate oxidase, urate oxidase, or ascorbate oxidase. In one embodiment, the enzyme includes at least one of laccase, bilirubin oxidase, tyrosinase, or polyphenol oxidase. In one embodiment, the conducting polymer includes at least one of polyaniline, polyp yrrole, polythiophene, poly(3,4-ethylenedioxythiophene), poly(p-phenylene sulfide), polyfluorine, polyphenylene, polypyrene, polyazulene, polynaphthalene, poly(acetylene), poly(p-phenylene vinylene), or polyphenyldiamine. In one embodiment, the carbon-based ink includes an enzymatic catalyst dispersed in the ink.

The circuit could also be touch sensitive, reacting to a user's press, (e.g. the circuit could be completed / activated when the user touches her finger on a particular section; the user's touch could light an LED, or prompt the circuit to collect / send data to the phone, etc). The capacitive touch sensor could be made from a conductive material (e.g. velostat) or capacitive glue. This could also conserve power and indicate to the user what her fertile status currently is.

The circuit could also be activated via an electrical pulse from another device such as a mobile phone.

To conserve the power source, the device could wait to activate sensing / calculations / or transmission until an event occurs physiologically or from the external device. The activation of the controller may also be initiated according to a pattern of predetermined time periods. Likewise, the sensor device 502 could activate the external device by transmitting information instead of only responding to requests from the external device. In one embodiment, the sensor device 502 includes a near-field communications (NFC) circuit that is powered by a magnetic field from an external device, such as cellular phone 100 for example. One advantage of the NFC circuit is that an internal power source, such as a battery for example, may be omitted. Upon receiving the initialization signal from the external device 100, the sensor device 502 may initiate measurements. Similarly, in another embodiment, upon receiving a signal from the sensor device 502, the external device 100 may initiate analysis of the measured data. In one embodiment, the sensor device 502 changes color in response to a signal from the external device 100.

In addition to triggering measurements based on time, triggers for taking measurements could also be done in conjunction with physiological / chemical changes such as heart rate that could help to determine when a user is sleeping or awake. These other physiological changes could not only allow us to adjust our measurement frequency but to also improve our estimations / calculations. For example, the heart rate values could be used to determine the time period during which the temperature values reflect BBT because we would be able to determine when the body is in a resting state. On the opposite side of the spectrum, we would be able to determine when the user is exercising or likely producing large quantities of sweat when the heart rate is high. We can then correlate heart rate with probable sweat quantities and use this information to increase the accuracy of our calculations and readings.

In still another embodiment, the sensor device 502 could be a gel, lotion or die that reacts to changes in fluids. The sensor device 502 reacts to fluids, such as but not limited to sweat, saliva, and vaginal mucus for example. In embodiments, the sensor device 502 may measure concentrations of lactic acid, acetic acid and urea in vaginal secretions. The sensor device 502 may further detect changes beyond pH and include other indicators, such as but not limited to lipids on the skin, skin thickness, short chain or long chain fatty acids, silica acid, N-acetyl-b-D-glucosamine, electrical admittance, molecular changes, sebum production, impedance, capacitance, and water retention for example. The measurement of these indicators may be via a spectrometer, such as a secondary device attached to the user's phone or via the phone's internal camera. The measurement made by the device may then transmit the data or measurement information to a remote computing device for further analysis as is described herein.

It should be appreciated that the gel, lotion or die may be used separately from other sensor devices 502 described above. In one embodiment, the gel, lotion or die may be applied to a sensor device 502, such as the printed electronic skin tattoo for example, and the combination of the gel, lotion or die with the sensor circuitry on the user's skin to measure the biomarker or physiological indicator. In one embodiment, a different gel, lotion or die may be applied to the sensor circuitry to measure different physiological indicators. For example, in the luteal phase, the gel, lotion or die may cooperate with the sensor circuitry to measure eccrine sweat, which in the follicular phase lipids are measured. In one embodiment, a different gel, lotion or die may be applied by the user depending on the stage of the user's menstrual cycle (e.g. gels that react to varying levels of pH in order to illustrate the occurrence of a rise or decrease in pH). In this way, the physiological indicator that is sensitive to that stage may be used to provide improved feedback to the user.

In one embodiment, the device is created with one of the following techniques: screen-printing, roll-to-roll printing, aerosol deposition, or inkjet printing technique. In this embodiment, the ink could be carbon-based ink that includes an enzymatic catalyst dispersed in the ink.

In one embodiment the cathode and the anode are configured on the substrate using at least one of a screen-printing, roll-to-roll printing, aerosol deposition, or inkjet printing technique. A method to fabricate the device (or an epidermal biofuel cell device), comprising: depositing an electrically conductive ink on an electrically insulative paper substrate to form an anode electrode and a cathode electrode adjacent to and separated from one another and conduit wires connecting to each of the anode and the cathode, the depositing including printing the ink on a first stencil placed over the paper substrate, the first stencil including a patterned region configured in a design of the anode, cathode, and conduit wires to allow transfer of the ink on the paper substrate, and the first stencil inhibiting transfer of the ink in areas outside the patterned region; curing the electrically conductive ink; depositing an electrically insulative ink on the paper substrate to form an insulative layer that exposes the anode electrode and the cathode electrode, the depositing including printing the electrically insulative ink on a second stencil placed over the paper substrate, the second stencil including a printing region configured in a second design to allow transfer of the ink on the paper substrate, the second stencil inhibiting transfer of the ink in areas outside the printing region; curing the electrically insulative ink; and depositing an adhesive layer on the insulative layer that exposes the anode electrode and the cathode electrode, the adhesive substrate formed of a flexible electrically insulative material structured to adhere to the skin of a user, wherein the paper substrate includes an upper layer and a base paper layer, the upper layer comprising a release agent coated on the base paper layer and structured to peel off to remove the paper substrate.

In one embodiment, the gel, lotion or die may change color on the user's skin. The gel, lotion or die may be applied with a translucent or transparent indicator strip that attaches the gel, lotion or die to the user's skin. Further, it is contemplated that gel, lotion or die may be used with another device or object that is inserted into the user's body, such as a like a tampon or an oral device (such as a thermometer), or is used for urinalysis.

As discussed herein, the user may be notified of their current fertility status by a change in color of an indicator, such as the aforementioned gel, lotion, die or strip for example. In one embodiment, the sensor may have materials including a stable mannitol-peroxide complex and a nontoxic organic compound which forms a colored oxidation product in the presence of oxygen released from the peroxide. In one embodiment, the nontoxic organic compound is guaiac. In one embodiment, the sensor may be similar to that described in United States Patent 3,406,015, the contents of which are incorporated by reference herein. It should be appreciated that device incorporating this sensor may be an oral device or a skin mounted device for example. Further, this sensor may be one of several sensors in the device. This sensor may cooperate with other sensors for detecting one of a physiological parameters measured in connection with determining the users current fertility status.

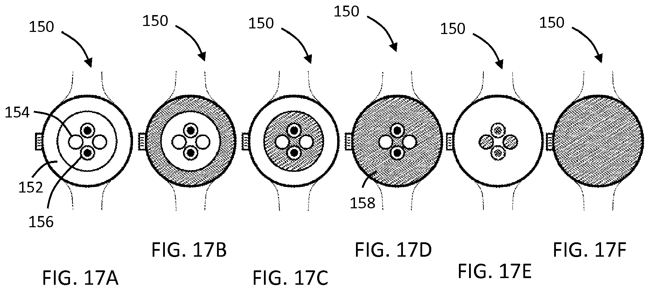

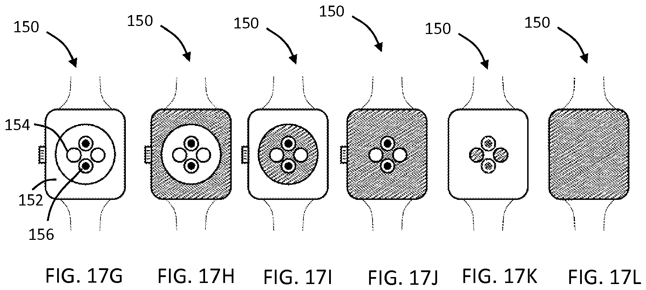

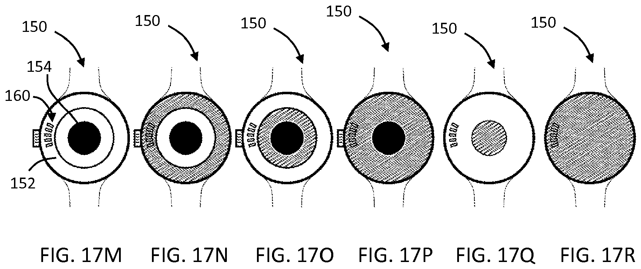

In addition to the previous embodiments, the device could be in the form of a garment or accessory or insertable / attachable component to a garment or accessory. The device could be strategically placed where there is more sweat or other parameters are in higher concentration, for example, underarm / armpit, underwire / bottom part of the breast, underwear, etc. One embodiment could be the device as a part of / as an attachment to / inserted into / adhered to a bra, e.g. a device that is in contact with the under part of the breast, attached to the underwire or part of the bra that touches the skin where sweat is more easily gathered, but is not in too high of a concentration that other parameters on the skin cannot be detected. In one embodiment the device integrated into or adhered to a watch or other wearable device, such as a so-called smart watch. As used herein, the term "smart watch" means a wearable device 150, such as one that may be worn on a user's wrist, having a processor responsive to executable instructions. The smart watch may include communications circuitry that is configured to transmit or receive signals from one or more external devices, such as a cellular phone for example. The device could be configured in such a way as shown in FIGS. 17A-17R where it covers the part of the watch or a wearable device that is against the skin without covering the other devices sensors or electronic components (i.e. blood pressure or pulse sensors) that are also integrated into the smart watch and need to be in contact with the user's skin to operate. In embodiments the device may be configured to extend around these components and adhere to the back of the smart watch/accessory.

The wearable device 150 may include a back surface 152 that is arranged adjacent the user's skin when worn. The surface 152 may include a plurality of sensors 154, 156 that are arranged to be in direct contact or be in close proximity to the user's skin. In one embodiment, the wearable device 150 may include wicking or hydrophilic material 158 (FIG. 17D for example) or moisture absorbent or hydrophilic materials. The wearable device 150 may also include a fluid transport system as described herein, either alone or in combination with the wicking material. The wearable device 150 may include a combination of sensors 154, 156. The wicking material 158 may operate on only a portion of the surface 152, such as the outer periphery (FIG. 17B), an inner area (FIG 17C). In one embodiment, the surface 152 may include a plurality of apertures or vent openings 160. The apertures 160 may allow fluids (e.g. eccrine sweat) to pass through to the sensors. In one embodiment, a wicking material may be disposed to transport fluids through the apertures 160. The wearable device may have a variety of form factors, such as a round shape (FIGS. 17A- 17F), square shape (FIG. 17G - 17L) for example. The sweat collection or fluid transport system may also serve as an indicator to the user or device that the sensor or entire device should be changed, disposed of, or recalibrated. In one embodiment, the wicking material could expand as it accumulates fluid and when the material expansion reaches a threshold as determined visually via the configuration of the device or as determined via a sensor such as a humidity sensor or ion concentration sensor for example, or as determined programmatically via the calculated sweat quantity over time, an action must be taken, for example to change, dispose of, or re-calibrate the sensor.

As has been discussed herein, the use of multiple sensors, such as sensors 154, 156, 160 for example, that measure multiple different biomarkers provides advantages in improving the reliability or accuracy of the device. In one embodiment, the different sensors may be used at different times during the user's cycle to predict a fertility status or conception risk. For example a pH or ion sensor may be used to measure bio markers in eccrine sweat to determine when the user enters the fertile window (e.g. higher risk/probability of conception). For example, a surge or rapid increase in pH or chloride from the user's baseline measurement at the start of the menstrual phase (this baseline is sometimes referred to as the "coverline", a common term used when discussing BBT especially). Then a measurement of the user's basel body temperature is monitored to find the lowest point (e.g an inflection point) in temperature during the fertile window (several days later) to mark the 2nd day before ovulation, peak fertility. In one embodiment, the determination of status may be validated by detecting a second surge in pH. By correlating the unrelated biomarkers, the device may assist the user in determining the time when she is most fertile (FIG. 3C). When a BBT trend shows a rapid increase in temperature, the device determines the user is in a time period right before ovulation. Then 24 - 48 hours later the device may indicate that the user is no longer in the fertile window. When the BBT trend measurements indicate a decline to a baseline value, the device may predict the beginning of menstruation.

It should be appreciated that all of the sensors do not need to be operated during the entire cycle. In one embodiment, the ion sensors are operated from the beginning of the cycle through ovulation and then disabled until the start of the next cycle. In another embodiment, the BBT sensor may operate once a rapid increase or surge in pH or chloride is detected.

In the event that the biomarker values do not return to a baseline value or otherwise indicate a change in health status, the device may adapt by monitoring different biomarkers or by changing the data model used to be appropriate for the condition detected. For example, if certain biomarkers such as progesterone do not return to baseline levels after the fertile window ends (or should have ended), then this may indicate that conception has occurred and the device may change to a different data model that is appropriate for pregnancy. In one embodiment, the biomarkers that are monitored may be user selected. For example, in the case where pregnancy is detected, the user may select to monitor ascorbic acid (Vitamin C) to reduce the risk of miscarriage.

In one embodiment, one of the sensors 54, 56, 60 is a skin thickness sensor, such as an echo density, ultrasound or piezo electric sensor for example, to determine the skin thickness value. It has been found that there is a correlation between skin thickness and fertility status. When the woman enters the fertile window the thickness of her skin increases from a baseline skin thickness at the start of the menstrual phase. The skin thickness value remains relatively the same throughout the fertile window and into the luteal phase so it is not a good indicator for ovulation and the end of the fertile window. However, when combined with another biomarker sensor, such as a BBT sensor that is operated after the fertile window is detected, the most fertile day (e.g. two days before ovulation at lowest point) and ovulation (sharp rise in temperature) may be determined. The skin thickness sensor may then be operated again when the skin thickness returns to the baseline value, which indicates the onset of menstruation. The benefits of sensing skin thickness as opposed to sweat analytes is because one can avoid the challenges of compensation for drift, calibration, changes in sweat rate, and sweat collection / removal.

The sensors used herein may be calibrated by the user prior to use or may be purchased in a calibrated state. In one embodiment, the sensors may be removable or replaceable from the wearable device 150. The sensors may also be modular, meaning that they include a standard interface that allow the sensors to be replaced with the same or a different sensor depending on the biomarkers to be monitored. The removed sensor may be replaced with a new sensor or may be recalibrated to accommodate changes in the sensor over time or use. In one embodiment, the calibration of the sensor is compensated based on the measurement of factors that affect the reliable measurement of values, such as time and temperature for example. For example, in the case of a pH sensor, some pH sensors exhibit drift over time based on the operating temperature. By tracking the usage and the environment in which the wearable device is operating, the drift effects may be compensated for without recalibration or allow for extended operating periods before recalibration. This behavior in one embodiment could be represented by a component data model based on the expected behavior of the component or sensor under different circumstances, environments or applications. This allows the determination of the users physical condition (e.g. fertility status) based not only on the population or personal data models but on one or more of the component data model as well.

The wearable device 150 may also include other external factor sensors that measure parameters not related to a biomarker, such as but not limited to an accelerometer, a heart rate sensor, a humidity sensor, a light sensor, a temperature sensor or an antenna for example, that allows the device to determine when the user is at a resting state. This provides advantages in measuring certain biomarkers, such activity may change the user's sweat rate or BBT. In one embodiment an environmental sensor, such as a humidity sensor for example, may also be used.

In one embodiment, the wearable device 150 may determine the fertility status based on a single reading by combining the measurements of multiple sensors and correlating these values to determine a fertility status at that instant in time. The use of multiple sensors to determine a plurality of biomarker values may include the use of a primary biomarker value in combination with a secondary biomarker value. Where the secondary biomarker provides confirmation of where the user is within her cycle. In other embodiments, the wearable device 150 may track the profile of the biomarker (e.g. a rapid increase in pH or ion values) and determines the fertility status based on the trend of the profile, either alone or in combination with the trending profiles of other biomarkers. It should be appreciated that while the wearable device 150 is illustrated as a device worn on the wrist, this is for exemplary purposes and other device form factors may also be used. In the embodiment that determines fertility based on a single reading, the device may be a portable device such as but not limited to a pen, a probe or another mobile device that is sized and shaped to be operated by a single person.

In one embodiment the device is in the form of a pad (e.g. sweat pad with elastic) or as an attachment to clothing that is in contact with the underarm or armpit where the highest amount of sweat can be found. The device could also have a secondary sensor point where there is less sweat so parameters that are found in direct contact with the epidermis could be tracked as well (e.g. two locations - armpit sensor + forearm sensor). In another embodiment, the device could also include a sensor or pad that is part of / inserted into / attached to a pair of underwear or similar to a tampon. This could be a standalone device or could be paired with a secondary device / sensor point (e.g. bra and underwear, or underarm (armpit pad) and forearm / top of shoulder) and the two devices would cooperate to collect different biomarkers or the same biomarkers to compare / contrast/ add to the list of parameters that are collected to determine fertile / hormonal levels.

These parameters could include - impedance / water retention, ph level, ion levels (e.g. chloride, potassium, sodium), hormonal levels (in underwear / tampon, e.g. luteinizing hormone, estrogen), viscosity of cervical fluid (underwear / tampon), temperature, urinary analysis, in any combination and from only a primary sensor point or in combination with a secondary sensor point or any number of other sensor points.

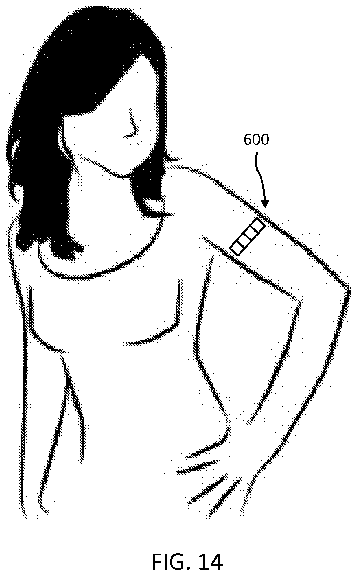

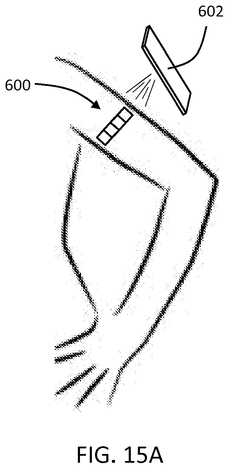

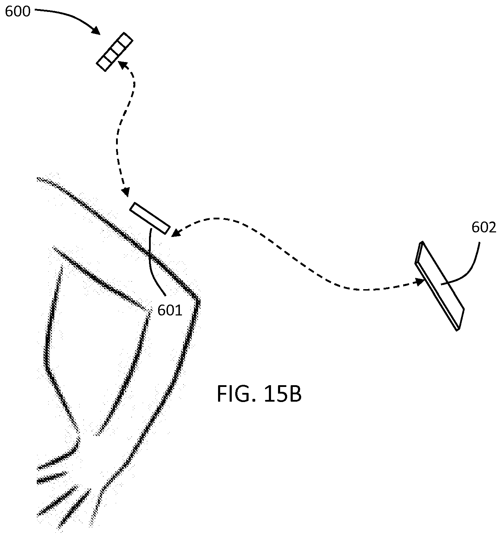

Referring now to FIG. 14, 15A and 15B another embodiment is shown of a sensor device 600. In this embodiment, the device 600 is adhesively attached to the user's body, such as an arm for example. In one embodiment, the device 600 is arranged to be in contact with the user's sweat. The device 600 may include one or more individual sensors that measure different properties, such as the pH, of the eccrine sweat. It has been found that the certain chemical or physical biomarkers (e.g. pH, conductivity, Na+, K+, NH+, Ca2+ and Cl-) of a woman's eccrine sweat changes during the course of her cycle with an initial peak pH reading right before the fertile window begins and then a second peak pH reading being measured a short period of time (e.g. a day) before ovulation. As discussed above, the ions may surge (e.g. rapidly increase in levels) prior to the fertile window and then again prior to ovulation. Further, in one embodiment the biomarkers from the eccrine sweat may be compared with or verified by comparing with other biomarkers, such as skin thickness or BBT for example.

In one embodiment, the device 600 is configured to change color based on measured pH. It should be appreciated that the sensor may be chemical based ion sensor that does not need electronics or a power source to operate. The pH may be determined by the user based on a scale printed onto the device 600, or the user may have a reference card with a color chart. In this embodiment, the user may compare the color of the sensor on device 600 with the chart to determine the corresponding pH. In one embodiment, the chemical sensor may be similar to a Reflectoquant® indicator strip manufactured by Merck KGaA, Darmstadt, Germany. The chemical sensor may also be a reagent type strip or a combination of the foregoing. It should be appreciated that not having a power supply or other electronics circuity may provide advantages in allowing usage of the device 600 in developing geographic regions that do not have the infrastructure to support an automated hormonal level monitoring system such as that which utilizes a cloud based architecture. In one embodiment, the pH scale on either the device 600 or the reference card is for a pH range from 4 - 6 in 0.05 or 0.1 increments. In one embodiment, the device 600 includes a body made from translucent material that allows the color of the sensor to be seen through the body. In one embodiment, the indicator on the sensor (colored strips) changes as a result of thermal changes on the skin.

In other embodiments, the device 600 may include a circuit having an ion sensitive field effect transistor (ISFET), such as a pH-ISFET or a CHEMFET sensor produced by D+T Microelectronicica, AIE, Bellaterra, Spain for example. In this embodiment, the circuit may include an indicator, such as a continuous spectrum LED or an OLED for example, that emits a colored light based on the measured pH (pH-ISFET) or Na+, K+, NH+, Ca²⁺ and Cl⁻ (CHEMFET) for example. In some embodiments, the ISFET sensor based device 600 may provide advantages in allowing a single device 600 to operate for months or provide communications to a computing device as discussed herein above. In the exemplary embodiment, the device 600 uses a non-glass sensor that does not require the use of a gel or liquid to facilitate contact with the user's skin.

It should be appreciated that the sensors described herein may also be made other materials that may not require electronic circuits or a power source. For example, the sensors may provide indications based on its chemical makeup and its reaction to external elements, including but not limited to chemical, thermal, light, ultra-violet, ionic or other reactions on the molecular level. In one embodiment, the sensors may be made from materials that are sometimes referred to as "smart materials", such as but not limited to include reversible chromism, ionochromism, electrochromism, photochromism, thermochromism , (e.g. topochemical polymerization of diacetylenes (DAs), self-layered polydiacetylene (PDAs), or functionalized with hydrazine bind) that could change and reverse color based on ionic changes in the fluids.

As discussed, this embodiment provides advantages in allowing for hormonal tracking even in less developed areas or with less sophisticated user's. However, it also allows for scalability and connection with a computing device, such as a cellular phone 602 (FIG. 15A). Since the device 600 communicates the measured value via a color, a camera may be used to acquire an image of the device 600 and the value determined via colorimetric analysis. The camera in computing device 602 is used as a colorimeter to determine the color of the sensor. It should be appreciated that a digital sensor on a camera may acquire wavelengths of light beyond the visible spectrum, allowing for a broader range of wavelengths of light to be used. In one embodiment, the device 600 may have multiple sensors that have different color scales, including non-visible colors that may be detected and parsed by the computing device using colorimetric analysis. Once the color is determined, the pH or other measured value may be determined. The computing device may then perform steps, such as those described in reference to FIGS. 7 -11 and 13 for storing and providing further analysis.

In another embodiment, shown in FIG. 15B, an intermediary device 601 is used to transfer data from the device 600 to an external computing device 602. In this embodiment, the device 600 includes a communications circuit for wirelessly transmitting data. In some embodiments, the communications circuit may be a low power circuit such as an active or passive RFID or NFC circuit. It should be appreciated that other types of communications circuits, including Wifi, WiGig or Bluetooth or transmission via capacitive coupling or electromagnetic induction may also be used. In still other embodiments, a capacitive coupler/ inductive coupler / electromagnetic induction may be used to provide a communication medium using the user's body. The communications circuit transmits the data to a nearby intermediary device 601. The intermediary device 601 may be a wearable device (i.e. a watch, bracelet, necklace, broach or pin) or an object that the user may keep in close proximity, such as a keychain for example. The intermediary device 601 includes communications circuit that is capable of receiving data from the device 600 using a first communications protocol (e.g. RFID or NFC) and communicates with the external computing device 602 using a second communications protocol (e.g. Bluetooth, Wifi or Wigig). The intermediary device provides advantages in allowing the external computing device 602 to receive data from the device 600 even if the external computing device 602 does not have a communications circuit compatible with the device 600. The intermediary device 601 also allows the device 600 to use a lower power (or passive) communications circuit that allows for conservation of the onboard electrical storage device. The intermediary device 601 may also allow for the collection of data at multiple points during the day. This data may be stored by the intermediary device 601 and transmitted to the external computing device 602 when it is available. In some embodiments, that intermediary device 601 may include an indicator that displays or indicates to the user their fertility status. In another embodiment, the primary device could store the collected data from multiple points in time and transmit the information to the external computing device when it is available.

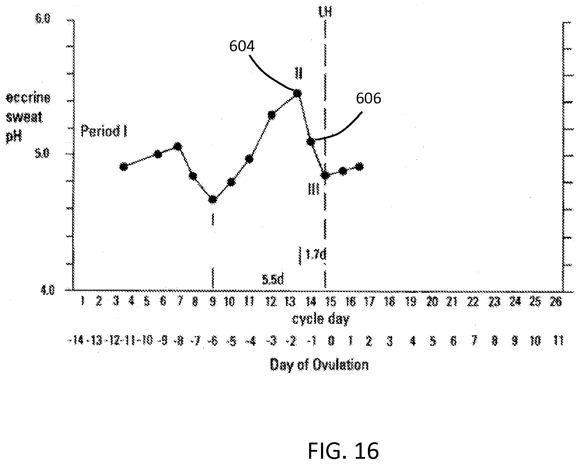

It should be appreciated that the device 600 may be a single use, a daily use, a cycle use or a multi-cycle use device. In a daily use embodiment, the user applies the device 600 to their skin and obtains the measured value (e.g. pH Na+, K+, NH+, Ca²⁺ and Cl⁻). The user uses a different device 600 each day. Using this value, such as by graphing the value over a period of time, the user may then is able to determine where they are in their cycle. For example, if the user sees a peak value 604 (FIG. 16) followed by a marked decrease 606, they will know that they are moving from the follicular phase into the ovulation stage. The user may then be able to take the desired steps to either inhibit or promote conception. It should be appreciated that the graphing may be done manually, or automatically by the computing device (e.g. via colorimetrics). Further in some embodiments, the computing device determines the change in phase and displays for the user a risk level as described herein above.

A single use device 600 is similar to the daily use embodiment except that the user may only use the device 600 on a periodic or aperiodic basis. In this embodiment, the user would determine a baseline for one or a set of biomarkers, for example a baseline range pH level (e.g. 4.5 - 5) for the portion of their cycle outside of the peak readings (e.g. 5.5) (e.g. taken during the menstruation phase). Then, when the user desires to know their fertility level, they use the single use device 600 to obtain a measured value or values for the set of biomarkers. By comparing this measured value to their baseline values, they may determine their fertility level.

A cycle and multi-cycle use device 600 is similar to the daily use device, except that the user will keep the device 600 on for the entire period of time. In the case of the cycle use device 600, the device 600 is discarded at the end of the cycle. In the multi-cycle embodiment, the device 600 is used for several cycles before being replaced. In some embodiments, the device 600 has a replaceable sensor that the user may exchange on a periodic or aperiodic basis. The replacement sensor may be a new sensor (e.g. the old sensor is discarded), or the existing sensor may be recalibrated and re-used, such as to correct for pH drift for example.

In still another embodiment, the user may be provided with a kit of devices 600. The kit includes a first plurality of devices 600 and a second plurality of devices 600. Each of the first plurality of devices 600 are configured to measure values during the follicular phase, while the second plurality of devices 600 are configured to measure values during the ovulation phase. In one embodiment, once the user's measured value (e.g. pH) falls below a baseline (e.g. 5.0), then they switch to the second plurality of devices 600. This embodiment provides advantages in that the sensor of device 600 may be more precisely configured to measure the desired values that correlate to that phase of the cycle. In one embodiment, the first plurality of devices 600 measures one type of value (e.g. Na+ or pH) and the second plurality of devices 600 measures a different value (e.g. potassium or conductivity). In another embodiment, the first and second plurality of devices 600 both measure the same value, but with different levels of sensitivity.

It should be appreciated that while embodiments herein refer to a device 600 that is adhesively coupled to the user's skin, this is for exemplary purposes and the claimed invention should not be so limited. In other embodiments, the device 600 may be simply pressed against the user's skin to make the measurement. In one embodiment, the device 600 is a "pen" that dispenses a reagent onto the user's skin. The reagent then changes color to allow the user to record the measurement either manually or via colorimetric analysis.

It should be appreciated that while specific sensors or sensor technologies may have been described herein, other sensors including those that measure values associated with glucose, LH, lactate, oxygen, carbon dioxide, ascorbic acid, free amino acid secretions, bicarbonate, testosterone, alcohol, lactic acid, urea, uric acid, ascorbic acid, folical stimulating hormone (FSH), estrogen, progesterone, testosterone, androgens, androstenedione, beta-human chorionic gonadotriphin (hCG) and any of the ions that may be found in human sweat, saliva or urine may also be used to monitor the aforementioned biomarkers.

According to one aspect of the invention, a device for determining hormonal level, which can be used to determine a condition, is provided. The device comprising: at least one sensor, the sensors configured to measure a physiologic parameter; an indicator; and a controller coupled to the at least one sensors and the indicator, the controller having a processor configured to execute computer readable instructions when executed on the processor for activating the indicator to indicate a hormonal level in response to receiving signals from the plurality of sensors.

According to another aspect of the invention, a device for determining hormonal level is provided. The device comprising: a plurality of sensors, each of the sensors configured to measure a different physiologic parameter, wherein the plurality of sensors is selected from a group comprising a basal temperature, a saliva electrolyte sensor, a saliva ion sensor, a blood flow sensor, a skin humidity sensor, a body skin temperature, a pH sensor, a CO2 sensor, a pH ion sensor (one or more in the following group: potassium (K⁺), ammonium (NH4⁺), calcium (Ca²⁺), chloride (Cl⁻), nitrate (N03) and sodium (Na⁺)), a heart rate / pulse sensor, lipid sensor, impedance sensor, lactic acid/lactate system sensor, free amino acid secretion sensor, and CO2 bicarbonate sensor; an indicator; and a controller coupled to the plurality sensors and the indicator, the controller having a processor configured to execute computer readable instructions when executed on the processor for activating the indicator to indicate a hormonal level in response to receiving signals from the plurality of sensors.

According to yet another aspect of the invention, a method of determining hormonal level is provided. The method comprising: measuring a plurality of physiologic parameters: providing a hormonal level model on a network server computer; training the hormonal level model based on values of the user and aggregate user data, as the user and aggregate users' data selected from a group comprising physiological data, hormonal data, demographic data, and cycle data; receiving at the network server computer over a network, the plurality of physiologic parameters; determining a hormonal level based at least in part on the hormonal level model and the plurality of physiologic parameters; and indicating to a user the determined hormonal level.

According to yet another aspect of the invention, a method of determining hormonal level is provided. The method comprising: measuring a plurality of physiologic parameters: providing a hormonal level model on a device; training the hormonal level model based on values of the user and aggregate user data, as the user data and aggregate user data selected from a group comprising: physiological data, hormonal data, demographic data, and cycle data; receiving on the device, the plurality of physiologic parameters; determining a hormonal level based at least in part on the hormonal level model and the plurality of physiologic parameters; and indicating to a user the determined hormonal level.

According to yet another aspect of the invention, a method of determining hormonal level is provided. The method comprising: measuring a plurality of physiologic parameters: providing a hormonal level model on an application on an external device; training the hormonal level model based on values of the user data and aggregate user data, the user data and aggregate user data selected from a group comprising: physiological data, hormonal data, demographic data, and cycle data; receiving on the application the plurality of physiologic parameters; determining a hormonal level based at least in part on the hormonal level model and the plurality of physiologic parameters; and indicating to a user the determined hormonal level.

According to yet another aspect of the invention, a system is provided. The system comprising: a device for determining hormonal level the device comprising: at least one sensor, the sensors configured to measure a physiologic parameter; an indicator; a controller coupled to the plurality sensors and the indicator, the controller having a processor configured to execute computer readable instructions when executed on the processor for transmitting at least the physiologic parameter in response to a signal from the at least one sensor; a network server computer; and an application executable by the network server computer, the application configured to implement a method, the method comprising: receiving at the network server computer over a network, data from the device, the data including at least the physiologic parameter; determining based on the data the hormonal level; and transmitting to the device the determined hormonal level. In accordance with yet another embodiment, any of the device described herein may include or exclude an indicator on the device. In some embodiments, the indicator may also be remotely located (i.e. on a wearable device, a cellular phone, a tablet or a computer screen) from the sensing device.

According to yet another aspect of the invention, a system is provided. The system comprising: a device for determining hormonal level the device comprising: at least one sensor, the sensors configured to measure a physiologic parameter; an indicator; a controller coupled to the plurality sensors and the indicator, the controller having a processor configured to execute computer read-able instructions when executed on the processor for transmitting at least the physiologic parameter in response to a signal from the at least one sensor; an application executable by the device, the application configured to implement a method, the method comprising: receiving data from the device, the data including at least the physiologic parameter; determining based on the data the hormonal level; and receiving and indicating the determined hormonal level.

According to yet another aspect of the invention, a system comprising: a device for determining hormonal level the device comprising: at least one sensor, the sensors configured to measure a physiologic parameter; an indicator; a controller coupled to the plurality sensors and the indicator, the controller having a processor configured to execute computer read-able instructions when executed on the processor for transmitting at least the physiologic parameter in response to a signal from the at least one sensor; an external computing device; and an application executable by the external device, the application configured to implement a method, the method comprising: receiving data from the device and at least one second device, the data including at least the physiologic parameter; determining based on the data the hormonal level; and transmitting to the device the determined hormonal level.

According to yet another aspect of the invention, a system is provided. The system comprising: a device for determining hormonal level comprising: at least one sensor, the sensors configured to measure a physiologic parameter; an indicator; a controller coupled to the singular or plurality sensors and the indicator, the controller having a processor configured to execute computer readable instructions when executed on the processor for automatically transmitting data in response to receiving a signal from the at least one sensor; and an external device selected from a group comprising a cellular phone, a tablet, a laptop computer, a desktop computer, a wearable device having a processor, and an appliance having a processor, the external device having a visual or audio indicator; and an application executable by the external device, the application configured to implement a method, the method comprising receiving the data.

According to yet another aspect of the invention, a system comprising: a device for determining hormonal level comprising: at least one sensor, the sensors configured to measure a physiologic parameter; an indicator; a controller coupled to the singular or plurality sensors and the indicator, the controller having a processor configured to execute computer readable instructions when executed on the processor for automatically transmitting data in response to receiving a signal from the at least one sensor; and an external device selected from a group comprising a cellular phone, a tablet, a laptop computer, a desktop computer, a wearable device having a processor, and an appliance having a processor, the external device having a visual or audio indicator; and an application executable by the external device, the application configured to implement a method, the method comprising receiving the data.

According to yet another aspect of the invention, a system is provided. The system comprising: a device for determining hormonal level comprising: at least one sensor, the sensors configured to measure a physiologic parameter; an indicator; a controller coupled to the singular or plurality sensors and the indicator, the controller having a processor configured to execute computer readable instructions when executed on the processor for automatically transmitting data in response to receiving a signal from the at least one sensor; and an external device selected from a group comprising a cellular phone, a tablet, a laptop computer, a desktop computer, a wearable device having a processor, and an appliance having a processor, the external device having a visual or audio indicator; and an application executable by the external device, the application configured to: selecting at least one second device, the at least one second device being configured to perform at least a portion of a hormonal level data and evaluation; automatically transmit data to the device and the at least one second device; automatically transmitting the data to the network server model; automatically transmitting data from the network server model; automatically transmitting data from the device and the at least one second device.

According to yet another aspect of the invention, a device for determining hormonal level is provided. The device comprising: a plurality of sensors, each of the sensors configured to measure a different physiologic parameter; an indicator; a controller coupled to the plurality sensors and the indicator, the controller having a processor configured to execute computer readable instructions when executed on the processor for substantially simultaneously measuring the physiologic parameters and activating the indicator to indicate a hormonal level in response to measuring the physiologic parameters.

Technical effects of embodiments of the invention include allowing a user to determine their current physical condition, such as fertility status. The determination of the fertility status may be used for either conception or contraceptive purposes. Further technical effects of embodiments of the invention include the collecting of user medical data to allow predictive or proactive intervention to correct or prevent medical issues or to track health or activity levels that vary with the physiologic changes.

It should be appreciated that while embodiments herein reference the use of the device for monitoring fertility levels or status, the device 30 and the disclosed methods may also be used to deduce health / reproductive / hormonal / metabolic / activity trends for the user and to prevent, diagnose and monitor treatment for conditions. In one embodiment, the device may be used to determine conception or implantation. Blood flow to the skin has been seen to increase with conception. In one embodiment the device may be used for personalized medicine, to monitor pre-natal care, patient monitoring, clinical trial monitoring, reproductive health monitoring, nutrition monitoring, for example vitamin and mineral level monitoring, outpatient monitoring, medication monitoring and other treatment monitoring, drug use monitoring, allergic reaction monitoring, overall health or nutritional panel, early warning signs and symptoms monitoring for conditions such as a heart attack or stroke, insulin monitoring, heart rate and breathing monitoring for infants and those who have recently experienced a concussion, fever monitoring, infection monitoring, vital sign monitoring, blood clot risk monitoring, post-injury or post-surgery monitoring, cancer marker monitoring, autonomic nervous system monitoring, breath monitoring (e.g. deep breathing, parasympathetic / sympathetic breathing, relaxation monitoring), stress level monitoring, and physical activity monitoring. In an embodiment, the measurement of the biomarkers or physiological parameters/factors is used to determine other potential health problems, such as the potential for a disease including but not limited to cervical or testicular cancer, polycystic ovarian syndrome (PCOS), Cystic Fibrosis, Diabetes, thyroid issues, hormonal imbalances, for example. These issues may be determined by the correlation of several different or unrelated biomarkers, the combination of which may indicate a health issue. Such as by monitoring levels of hyperplasia (proliferation of cells) in the apocrine sweat on the breast, for example. The monitoring of biomarkers or physiologic parameter levels may also aid in the tracking of the onset of menopause, perimenopause and postmenopause, onset of cancer and the tracking of changes in pregnancy, including the onset of labor. It has been found that the onset of labor and oocyte maturation can be detected by analyzing estriol concentration in aa body fluid. It has also been found that estrogenic loss reduces the number of blood vessels in the skin with a lack of nutrients traveling to the skin's surface. Still further embodiments use the device 40 to track the health, fertility, hormonal status of women who are pre / post reproductive or pregnant / post-pregnancy, cycle irregularities, other illnesses or infections (e.g. cystic fibrosis, ovarian cancer). Further, while embodiments of the invention make reference to the use of the device with women, the claimed invention should not be so limited. In other embodiments, the device may be used with men to determine hormonal or health trends. Still further embodiments utilize the device with mammals for determining fertility or health status of the mammal, such as for breeding and agricultural purposes.

Changes in menstrual flow and duration, and cycle length have been used to determine whether a woman is in different stages of menopause.

The sensed biomarker combinations could be used to take a snapshot of the health of the individual and create a panel of results.

The device may include sensors that may be used to diagnose nutrient deficiencies or monitor nutrition. Nutrients can include macronutrients, carbohydrates, proteins, amino acids, fats, alcohol, minerals, vitamins, and water. The device could monitor levels of nutrients themselves in bodily fluids such as sweat, saliva, urine, semen, and cervical fluid. The device may also include sensors to monitor metabolites or metabolic activity. In another embodiment, the device could measure biomarkers whose levels or behavior correlate to nutrient levels. These measurements may be measured and analyzed to determine a nutritional panel or nutrient state including deficiencies, excesses, and imbalances. For example electrolyte imbalances, can be a symptom of nutrient deficiencies or menopause among others. This information in combination with data models such as the personal data models and population data models and a nutritional data model, could assist in the further analysis of the source of an imbalance, signifying health, conditions, or stages, e.g. menopause or more specifically a lack of a vitamin or mineral. This would be helpful to men and women in order to diagnose imbalances, monitor nutrient intake, monitor physical activity, and other health states and conditions. Proper iron levels for example are important for women who are more prone to anemia during menstruation and during pregnancy which can lead to serious complications. Sensors to detect nutrition could also include sensors to detect specific vitamins and minerals, such as iron, zinc, magnesium, calcium, manganese, phosphorus, potassium, sodium, vitamin C, and any other vitamins and nutrients that can be detected directly or indirectly in bodily fluids.

Further, while embodiments herein have described embodiments of the invention with reference to women's health or fertility, the claimed invention should not be so limited. In other embodiments, the sensor could be used to determine male fertility. In one embodiment, the sensor may detect in fluids (e.g. saliva, urine, semen, or sweat) compounds or ions that reflect the ingesting of foods or medicines that may change sperm production. For example, the sensor may detect the presence of proteins that indicate papain (papaya proteinase I), which may result from the eating of papaya seeds. It has been found that male mammals that eat papaya seeds decreases the production of semen and may render them temporarily infertile over time.

As will be appreciated by one skilled in the art, aspects of the present invention may be embodied as a system, method or computer program product, as shown in FIGs. 7 - 11. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) 602 having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read- only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages, or a scripting language such as Python. The program code may execute entirely on the user's device, cellular phone, wearable device or computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the Figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

The flow diagrams depicted herein are just one example. There may be many variations to this diagram or the steps (or operations) described therein without departing from the spirit of the invention. For instance, the steps may be performed in a differing order or steps may be added, deleted or modified. All of these variations are considered a part of the claimed invention.

While the invention has been described in detail in connection with only a limited number of embodiments, it should be readily understood that the invention is not limited to such disclosed embodiments. Rather, the invention can be modified to incorporate any number of variations, alterations, substitutions or equivalent arrangements not heretofore described, but which are commensurate with the spirit and scope of the invention. Additionally, while various embodiments of the invention have been described, it is to be understood that aspects of the invention may include only some of the described embodiments. Accordingly, the invention is not to be seen as limited by the foregoing description, but is only limited by the scope of the appended claims.

## Claims

1. A device (30, 150, 502, 600) for determining a user's physical condition, comprising:
a plurality of sensors (48, 54, 56) configured to measure a plurality of biomarker values associated with a user, each of the plurality of sensors (48, 54, 56) configured to measure a different biomarker value; and
a controller (50,38) operably coupled to the plurality of sensors (48, 54, 56), the controller (50,38) having a processor (106) and memory (108), the processor (106) configured to execute computer readable instructions when executed on the processor (106) for comparing a data model stored in the memory (108) associated with each of the biomarker values and determining the user's physical condition in response to receiving at least one signal from one of the plurality of sensors (48, 54, 56), wherein the data model includes one or more of a population data model, a personal data model, and a component data model, **characterized in that**
the device (30, 150, 502, 600) is configured such that, in the event that the biomarker values indicate a change in health status, the device (30, 150, 502, 600) can adapt to monitor different biomarkers or change the data model used.

2. The device of claim 1 wherein the processor (106) is further responsive to executable computer instructions when executed on the processor (106) for storing in memory (108) at least one of the biomarker values at a plurality of time periods, the processor (106) further being responsive for comparing a trend profile of the biomarker values stored in memory (108) to the data model to determine a fertility status.

3. The device of claim 1 wherein at least one of the plurality of sensors (48, 54, 56) is removably coupled to the device.

4. The device of claim 1 further comprising a fluid transport system disposed between at least one of the plurality of sensors (48, 54, 56) and the user's skin.

5. The device of claim 1 further comprising a housing (32), the plurality of sensors (48, 54, 56) coupled to the housing (32), wherein the housing (32) is sized and shaped to be worn on a portion of a body of the user or to be coupled to an article of clothing.

6. The device of claim 1 wherein at least one of the plurality of sensors (48, 54, 56) measures an external factor value.

7. A method of measuring biomarkers of a user, the method comprising:
providing a device according to any one of claims 1 to 6; and
measuring a plurality of biomarker values on the user with the plurality of sensors (48, 54, 56) of said device.

8. The method of claim 7 further comprising:
measuring a first biomarker value with a first sensor of the plurality of sensors (48, 54, 56) during a first time period; and
measuring a second biomarker value with a second sensor of the plurality of sensors (48, 54, 56) during a second time period.

9. The method of claim 7 further comprising contacting the plurality of sensors (48, 54, 56) to the user's skin.

## Patentansprüche

1. Vorrichtung (30, 150, 502, 600) zum Bestimmen des körperlichen Zustands eines Benutzers, die Folgendes umfasst:
eine Vielzahl von Sensoren (48, 54, 56), die dazu ausgelegt sind, eine Vielzahl von Biomarkerwerten, die mit einem Benutzer verknüpft sind, zu messen, wobei jeder der Vielzahl von Sensoren (48, 54, 56) dazu ausgelegt ist, einen anderen Biomarkerwert zu messen; und
eine Steuerung (50, 38), die an die Vielzahl von Sensoren (48, 54, 56) wirkgekoppelt ist, wobei die Steuerung (50, 38) einen Prozessor (106) und einen Speicher (108) aufweist, wobei der Prozessor (106) dazu ausgelegt ist, computerlesbare Anweisungen zum Vergleichen eines Datenmodells, das im Speicher (108) gespeichert ist, der mit jedem der Biomarkerwerte verknüpft ist, und Bestimmen des körperlichen Zustands des Benutzers in Reaktion auf das Empfangen von mindestens einem Signal von einem der Vielzahl von Sensoren (48, 54, 56) auszuführen, wenn sie auf dem Prozessor (106) ausgeführt werden, wobei das Datenmodell eines oder mehreres von einem Populationsdatenmodell, einem persönlichen Datenmodell und einem Komponentendatenmodell beinhaltet, **dadurch gekennzeichnet, dass**
die Vorrichtung (30, 150, 502, 600) derart ausgelegt ist, dass sich in dem Fall, in dem die Biomarkerwerte eine Änderung des Gesundheitsstatus anzeigen, die Vorrichtung (30, 150, 502, 600) zum Überwachen von anderen Biomarkern oder zum Ändern des verwendeten Datenmodells anpassen kann.

2. Vorrichtung nach Anspruch 1, wobei der Prozessor (106) ferner auf ausführbare Computeranweisungen zum Speichern von mindestens einem der Biomarkerwerte in einer Vielzahl von Zeitperioden im Speicher (108), wenn sie auf dem Prozessor (106) ausgeführt werden, reagiert, wobei der Prozessor (106) ferner auf das Vergleichen eines Trendprofils der Biomarkerwerte, das im Speicher (108) gespeichert ist, mit dem Datenmodell reagiert, um einen Fruchtbarkeitsstatus zu bestimmen.

3. Vorrichtung nach Anspruch 1, wobei mindestens einer der Vielzahl von Sensoren (48, 54, 56) entfernbar an die Vorrichtung gekoppelt ist.

4. Vorrichtung nach Anspruch 1, die ferner ein Fluidtransportsystem umfasst, das zwischen mindestens einem der Vielzahl von Sensoren (48, 54, 56) und der Haut des Benutzers angeordnet ist.

5. Vorrichtung nach Anspruch 1, die ferner ein Gehäuse (32) umfasst, wobei die Vielzahl von Sensoren (48, 54, 56) an das Gehäuse (32) gekoppelt sind, wobei das Gehäuse (32) dimensioniert und geformt ist, um an einem Abschnitt eines Körpers des Benutzers getragen oder an ein Kleidungsstück gekoppelt zu werden.

6. Vorrichtung nach Anspruch 1, wobei mindestens einer der Vielzahl von Sensoren (48, 54, 56) den Wert eines externen Faktors misst.

7. Verfahren zum Messen von Biomarkern eines Benutzers, wobei das Verfahren Folgendes umfasst:
Bereitstellen einer Vorrichtung gemäß einem der Ansprüche 1 bis 6 und
Messen einer Vielzahl von Biomarkerwerten am Benutzer mit der Vielzahl von Sensoren (48, 54, 56) der Vorrichtung.

8. Verfahren nach Anspruch 7, das ferner Folgendes umfasst:
Messen eines ersten Biomarkerwerts mit einem ersten Sensor der Vielzahl von Sensoren (48, 54, 56) während einer ersten Zeitperiode und
Messen eines zweiten Biomarkerwerts mit einem zweiten Sensor der Vielzahl von Sensoren (48, 54, 56) während einer zweiten Zeitperiode.

9. Verfahren nach Anspruch 7, das ferner das Verbinden der Vielzahl von Sensoren (48, 54, 56) mit der Haut des Benutzers umfasst.

## Revendications

1. Dispositif (30, 150, 502, 600) destiné à déterminer une condition physique d'un utilisateur, comprenant :
une pluralité de capteurs (48, 54, 56) configurés pour mesurer une pluralité de valeurs de biomarqueur associées à un utilisateur, chacun de la pluralité de capteurs (48, 54, 56) configuré pour mesurer une valeur de biomarqueur différent ; et
un moyen de commande (50, 38) couplé de façon opérationnelle à la pluralité de capteurs (48, 54, 56), le moyen de commande (50, 38) présentant un processeur (106) et une mémoire (108), le processeur (106) configuré pour exécuter des instructions lisibles par ordinateur lorsqu'exécutées sur le processeur (106) pour comparer un modèle de données stocké dans la mémoire (108) associé à chacune des valeurs de biomarqueur et déterminer la condition physique d'un utilisateur en réponse à la réception d'au moins un signal de l'un de la pluralité de capteurs (48, 54, 56), dans lequel le modèle de données inclut un ou plusieurs parmi un modèle de données démographiques, un modèle de données personnelles et un modèle de données de composante, **caractérisé en ce que**
le dispositif (30, 150, 502, 600) est configuré de telle sorte que, dans le cas où les valeurs de biomarqueur indiquent un changement de l'état de santé, le dispositif (30, 150, 502, 600) peut s'adapter pour piloter des biomarqueurs différents ou modifier le modèle de données utilisé.

2. Dispositif selon la revendication 1, dans lequel le processeur (106) est en outre réactif à des instructions d'ordinateur exécutables lorsqu'exécutées sur le processeur (106) pour stocker en mémoire (108) au moins l'une des valeurs de biomarqueur à une pluralité de périodes de temps, le processeur (106) étant en outre réactif pour comparer un profil de tendance des valeurs de biomarqueur stockées en mémoire (108) au modèle de données pour déterminer un état de fertilité.

3. Dispositif selon la revendication 1, dans lequel au moins l'un de la pluralité de capteurs (48, 54, 56) est couplé de façon amovible au dispositif.

4. Dispositif selon la revendication 1, comprenant en outre un système de transport de fluide disposé entre au moins l'un de la pluralité de capteurs (48, 54, 56) et la peau d'un utilisateur.

5. Dispositif selon la revendication 1, comprenant en outre un logement (32), la pluralité de capteurs (48, 54, 56) couplés au logement (32), dans lequel le logement (32) est dimensionné et façonné pour être porté sur une partie d'un corps de l'utilisateur ou pour être couplé à un article vestimentaire.

6. Dispositif selon la revendication 1, dans lequel au moins l'un de la pluralité de capteurs (48, 54, 56) mesure une valeur de facteur externe.

7. Procédé de mesure de biomarqueurs d'un utilisateur, le procédé comprenant :
la fourniture d'un dispositif selon l'une quelconque des revendications 1 à 6 ; et
la mesure d'une pluralité de valeurs de biomarqueur sur l'utilisateur avec la pluralité de capteurs (48, 54, 56) dudit dispositif.

8. Procédé selon la revendication 7, comprenant en outre :
la mesure d'une première valeur de biomarqueur avec un premier capteur de la pluralité de capteurs (48, 54, 56) pendant une première période de temps ; et
la mesure d'une seconde valeur de biomarqueur avec un second capteur de la pluralité de capteurs (48, 54, 56) pendant une seconde période de temps.

9. Procédé selon la revendication 7, comprenant en outre la mise en contact la pluralité de capteurs (48, 54, 56) avec la peau d'un utilisateur.
